# EUROPEAN PATENT SPECIFICATION

(11) **EP 1 872 726 B1**
(45) Date of publication and mention of the grant of the patent: **08.12.2010**
(21) Application number: 07252652.8
(22) Date of filing: 29.06.2007
(51) Int. Cl.: A61B 10/02, A61B 17/32

(54) **Energy biopsy device for tissue penetration and hemostasis**
Energiebiopsievorrichtung zur Gewebedurchdringung und Blutstillung
Dispositif de biopsie d'énergie pour pénétration de tissus et hémostase

(30) Priority: 30.06.2006 US 428033
(43) Date of publication of application: 02.01.2008
(73) Proprietor: ETHICON ENDO-SURGERY, INC., Cincinnati, Ohio 45242 (US)
(72) Inventor: Monson, Gavin M., Oxford Ohio 45056 (US); Hibner, John A., Mason Ohio 45040 (US); Stulen, Foster B., Mason Ohio 45040 (US); Weikel, Robert F., Jr., Hamilton Ohio 45011 (US)
(74) Representative: Tunstall, Christopher Stephen

(56) References cited:
- EP-A- 1 410 764
- WO-A-00/48519
- US-A- 5 397 293
- US-A- 6 117 152
- US-A1- 2003 229 293
- US-B1- 6 432 064

## Description

### Field of the Invention

The present invention relates, in general, to a method of imaging assisted tissue sampling and, more particularly, to an improved biopsy probe with an energy based tissue penetration system to pierce hard tissues, remove lesions, improve hemostasis, and provide greater tissue access.

### Background of the Invention

Core biopsy devices have been combined with imaging technology to better target a lesion in breast tissue. One such commercially available product is marketed under the trademark name MAMMOTOME^{™}, by Ethicon Endo-Surgery, Inc. An embodiment of such a device is described in U.S. Patent No. 5,526,822 issued to Burbank, et al., on June 18, 1996. Its handle receives mechanical and electrical power as well as vacuum assist from a remotely positioned control module that is spaced away from the high magnetic field of a Magnetic Resonance Imaging (MRI) machine.

As seen from that reference, the instrument is a type of image-guided, percutaneous coring, breast biopsy instrument. It is vacuum-assisted, and some of the steps for retrieving the tissue samples have been automated. The physician uses this device to capture "actively" (using the vacuum) the tissue prior to severing it from the body. This allows the sampling of tissues of varying hardness. In addition, a side opening aperture is used, avoiding having to thrust into a lesion, which may tend to push the mass away. The side aperture may be rotated about a longitudinal axis of the probe, thereby allowing multiple tissue samples without having to otherwise reposition the probe. These features allow for substantial sampling of large lesions and complete removal of small ones. Handheld breast biopsy instruments are also available that allow the physician to perform the tissue penetration and probe placement manually.

Tissue penetration into the breast is accomplished with a surgical sharp at a distal end of the breast biopsy instrument. The surgical sharp cuts and pushes into tissue and penetration forces can be high, particularly when attempting to penetrate hard or dense lesions.

Insertion forces can be reduced if the breast biopsy device uses means other than a surgical sharp to create a passage or tunnel as it is inserted, and dense lesions could be penetrated without being pushed away. Energy delivery devices such as ultrasound, RF, thermal heaters, and lasers are used to tunnel passageways, cut tissue, and provide reduced penetration forces. Energy delivery devices can also provide improved hemostasis, and when combined with a biopsy system such as that described above, offer useful advantages when applied to other biopsy modalities such as prostate kidney, liver, lung, uterus and the like.

By way of example, U.S. Pat. No. 6,274,963 to Eastabrook et al., an ultrasonic handle or handpiece is disclosed that may be used to penetrate, cut, and coagulate tissue. EP-A-1 410 764 discloses a biopsy device according to the preamble of claim 1 comprising an elongated needle with a needle tip and an ultrasonic cutting tip snapped onto the needle tip to assist insertion of the biopsy device into a body.

Additionally, some breast lesions can be located in difficult places in a patient, such as next to a rib. By using energy delivery devices in combination with a biopsy system, the length of the surgical sharp could be eliminated, providing a greater range of access to difficult surgical sites. Additionally, the energy delivery device at the distal tip could be used to ablate or cauterize lesion tissue, rather than removing it. Consequently, a significant need exists for a biopsy system with reduced penetration forces, improved tissue lesion penetration, better tissue access, elimination of a surgical sharp from the operating room, and improved hemostasis capabilities.

### Brief Summary of the Invention

The present invention provides a biopsy system as claimed hereinafter. The invention overcomes the above-noted and other deficiencies of the prior art by providing a biopsy system that includes an energy based tissue penetration system that eliminates a surgical sharp, reduces tissue penetration forces, can penetrate dense or hard tumors, provides increased access to difficult surgical sites, offers increases hemostasis, cauterizes or ablates tissue, and can offer features useful in taking biopsies in body tissue other than breast. With such a system, the surgeon can have the full functionality of vacuum assisted core biopsy systems with additional energy enhancements that increase the usefulness of the system and provide surgeon benefits.

These and other objects and advantages of the present invention shall be made apparent from the accompanying drawings and the description thereof.

### Brief Description of the Figures

The accompanying drawings, which are incorporated in and constitute a part of this specification, illustrate embodiments of the invention, and, together with the general description of the invention given above, and the detailed description of the embodiments given below, serve to explain the principles of the present invention.
FIGURE 1 is a perspective disassembled view of a Magnetic Resonance Imaging (MRI) biopsy system including a handpiece ("biopsy device") having intuitive graphical controls consistent with aspects of the invention.
FIGURE 2 is an isometric view of a lateral fence and pedestal of a localization fixture of the MRI biopsy system of FIG. 1.
FIGURE 3 is an isometric view of a guidance assembly mounted on a right primary targeting rail of FIG. 2.
FIGURE 4 is an exploded isometric view of the guidance assembly of FIG. 3 and the sleeve trocar and introducer obturator of FIG. 1.
FIGURE 5 is an isometric view of an introducer obturator not in accordance with the invention inserted into the sleeve trocar of FIGS. 1 and 4.
FIGURE 6 is an aft right isometric view of the MRI biopsy device of FIG. 1 with a disposable probe assembly and keypad control disengaged from a reusable holster portion.
FIGURE 7 is a fore left isometric view of the MRI biopsy device of FIG. 1 with the disposable probe assembly and keypad control disengaged from the reusable holster portion.
FIGURE 8 is a fore left exploded isometric view of the reusable holster portion of FIG. 7.
FIGURE 9 is a top view of the disposable probe assembly of FIG. 7 with an upper cover removed to expose interior components of a carriage cavity.
FIGURE 10 is a fore left exploded isometric view of the disposable probe assembly of FIG. 7.
FIGURE 11 is an aft left isometric view of the localization fixture and guidance assembly installed into a breast coil of FIG. 1.
FIGURE 12 is an aft isometric view of the MRI biopsy device of FIG. 7 into the guidance assembly of FIG. 11.
FIGURE 13 is a top detail view of a display portion of the MRI biopsy device of FIG. 7.
FIGURE 14 is an aft right isometric view of the MRI biopsy device, localization fixture and breast coil of FIG. 12 with insertion of a marker deploying instrument through a probe of the disposable probe assembly.
FIGURE 15 is a perspective disassembled view of the biopsy system of FIG. 1 including an ultrasonic tissue penetration system.
FIGURE 16 is an aft isometric view of the MRI biopsy device of FIG. 12 with an ultrasonic tissue penetration system placed into the guidance assembly of FIG. 11 and penetrating tissue.
FIGURE 17 is a side view of the ultrasonic tissue penetration system assembly of FIG. 15 showing a section view of the ultrasonic handpiece assembly including a sleeve trocar.
FIGURE 18 is a cross section of a shaft and sleeve trocar of the ultrasonic handpiece assembly of FIG. 17.
FIGURE 19 is an isometric side view of the ultrasonic handpiece assembly of FIG. 17 showing the shaft elements exploded.
FIGURE 20 is a cross sectional view of an ultrasonically active distal tip of the ultrasonic handpiece assembly of FIG. 17 with a sleeve trocar as it tunnels into breast tissue and a hard tumor.
FIGURE 21 is an alternate cross sectional view of FIG. 20 with the ultrasonic distal tip removed from the sleeve trocar, the MRI biopsy device of FIG. 1 inserted, and a portion of the hard tumor drawn into a probe of the MRI biopsy device.
FIGURE 22 is an alternate cross sectional view of FIG. 21 with the MRI biopsy device of FIG. 1 inserted, and a cutter tube of the MRI biopsy device partially severing a tumor drawn into the MRI biopsy device.
FIGURE 23 is an alternate cross sectional view of FIG. 22 with the MRI biopsy device of FIG. 1 inserted, and a cutter tube of the MRI biopsy device fully severing a tumor drawn into the MRI biopsy device.
FIGURES 24a-f are a series of isometric views showing a number of different distal tip configurations.
FIGURE 25 is an isometric view show a handheld surgical biopsy system ready to be combined with an ultrasonic penetration system.
FIGURE 26 is an isometric view of the handheld surgical biopsy system of FIG. 25 showing a first variant of the ultrasonic penetration system ready to be inserted in a generally continuous passageway of the handheld surgical biopsy system.
FIGURE 27 is an isometric view of the handheld surgical biopsy system of FIG. 25 showing a second variant of the ultrasonic penetration system ready to be inserted in a generally continuous passageway of the handheld surgical biopsy system.
FIGURE 28 is across sectional view of an end effector of the handheld surgical biopsy system and ultrasonic penetration system of FIG 26 as it tunnels into breast tissue.
FIGURE 29 is across sectional view of an end effector of the handheld surgical biopsy system of FIG. 26 with the ultrasonic penetration system removed and a portion of the hard tumor drawn into a side aperture and being severed.
FIGURE 30 is across sectional view of an an alternate end effector of a surgical biopsy system with an ultrasonic penetration and cauterization system attached to a distal end of the surgical biopsy system.
FIGURE 31 is across sectional view of an an alternate end effector of a surgical biopsy system with a bipolar RF penetration and cauterization system attached to a distal end of the surgical biopsy system.

### Detailed Description of the Invention

A biopsy device advantageously includes an energy delivery system such as an ultrasonic tissue penetration system to reduce tissue penetration forces, improve penetration and excision of hard tumors, and improve hemostasis. Additionally, the ultrasonic tissue penetration system improves lateral biopsy port access by eliminating the added length of the surgical sharp from a penetrating end of a biopsy device and moving the lateral biopsy port closer to the penetrating end providing increased tissue access. Energy based penetration systems can also ablate hard to reach tissue and provide improved hemostasis and can address bleeders. A lateral biopsy port can be blocked to prevent tissue damage from contact with active elements of the energy delivery system. The energy delivery system can be adapted for use with a variety of biopsy systems including a MRI biopsy device and a handheld biopsy device.

### MRI Biopsy Device

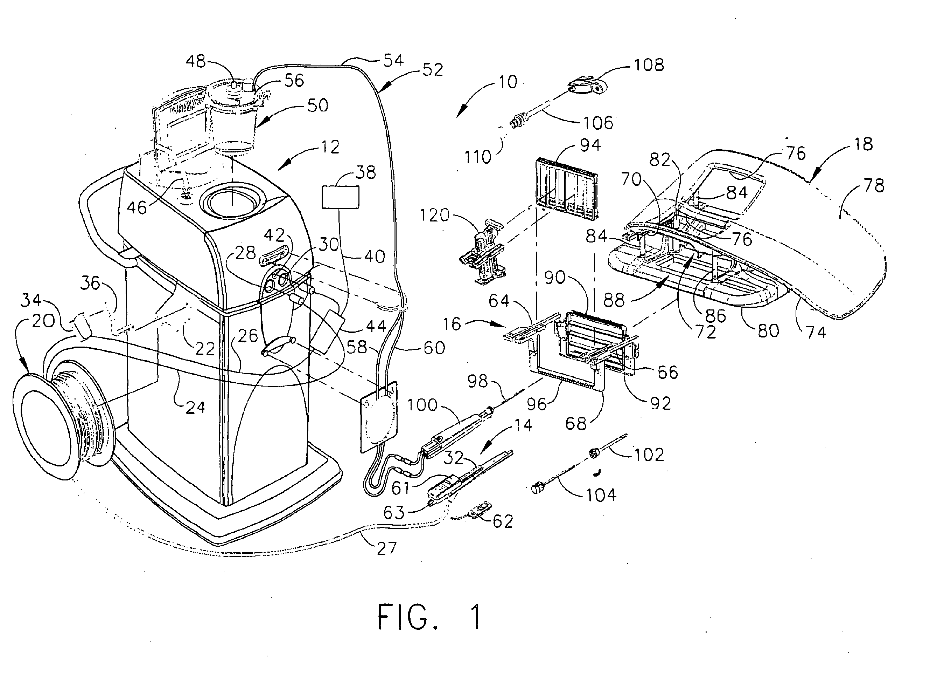

Turning to the Drawings, wherein like numerals denote like components throughout the several views, in FIGS. 1-3, a Magnetic Resonance Imaging (MRI) compatible biopsy system 10 has a control module 12 that typically is placed outside of a shielded room containing an MRI machine (not shown) or at least spaced away to mitigate detrimental interaction with its strong magnetic field and/or sensitive radio frequency (RF) signal detection antennas. As described in U.S. Pat. No. 6,752,768. a range of preprogrammed functionality is incorporated into the control module 12 to assist in taking these tissue samples. The control module 12 controls and powers an MRI biopsy device ("handpiece") 14 that is positioned and guided by a localization fixture 16 attached to a breast coil 18 that is placed upon a gantry (not shown) of the MRI machine.

A cable management spool 20 is placed upon a cable management attachment saddle 22 that projects from a side of the control module 12. Wound upon the cable management spool 20 is a paired electrical cable 24 and mechanical cable 26 which are bundled into sheathed cable 27 for communicating control signals and cutter rotation/advancement motions respectively. In particular, electrical and mechanical cables 24, 26 each have one end connected to respective electrical and mechanical ports 28, 30 in the control module 12 and another end connected to a reusable holster portion 32 of the MRI biopsy device 14. An MRI docking cup 34, which may hold the holster portion 32 when not in use, is hooked to the control module 12 by a docking station mounting bracket 36.

An interface lock box 38 mounted to a wall provides a tether 40 to a lockout port 42 on the control module 12. The tether 40 is advantageously uniquely terminated and of short length to preclude inadvertent positioning of the control module 12 too close to the MRI machine. An in-line enclosure 44 may advantageously register the tether 40, electrical cable 24 and mechanical cable 26 to their respective ports 42, 28, 30 on the control module 12.

Vacuum assist is provided by a first vacuum line 46 that connects between the control module 12 and an outlet port 48 of a vacuum canister 50 that catches liquid and solid debris. A tubing kit 52 completes the pneumatic communication between the control module 12 and the MRI biopsy device 14. In particular, a second vacuum line 54 is connected to an inlet port 56 of the vacuum canister 50. The second vacuum line 54 divides into two vacuum lines 58, 60 that are attached to the MRI biopsy device 14. With the MRI biopsy device 14 installed in the holster portion 32, the control module 12 performs a functional check. Saline is manually injected into biopsy device 14 to serve as a lubricant and to assist in achieving a vacuum seal. The control module 12 actuates a cutter mechanism (not shown) in the MRI biopsy device 14, monitoring full travel. Binding in the mechanical cable 26 or within the biopsy device 14 is monitored with reference to motor force exerted to turn the mechanical cable 26 and/or an amount of twist in the mechanical cable 26 sensed in comparing rotary speed or position at each end of the mechanical cable 26.

Just proximal to a display area 61 on the reusable holster portion 32, a remote keypad 62, which is detachable from the reusable holster portion 32, communicates via the electrical cable 24 to the control module 12 to enhance clinician control of the MRI biopsy device 14, especially when controls that would otherwise be on the MRI biopsy device 14 itself are not readily accessible after insertion into the localization fixture 16 and/or placement of the control module 12 is inconveniently remote (e.g., 30 feet away). An aft end thumbwheel 63 on the reusable holster portion 32 is also readily accessible after insertion to rotate the side from which a tissue sample is to be taken.

Left and right parallel upper guides 64, 66 of a localization framework 68 are laterally adjustably received respectively within left and right parallel upper tracks 70, 72 attached to an under side 74 and to each side of a selected breast aperture 76 formed in a patient support platform 78 of the breast coil 18. A base 80 of the breast coil 18 is connected by centerline pillars 82 that are attached to the patient support platform 78 between the breast apertures 76. Also, a pair of outer vertical support pillars 84, 86 on each side spaced about a respective breast aperture 76 respectively define a lateral recess 88 within which the localization fixture 16 resides.

In FIGS. 1-2, a selected breast is compressed along an inner (medial) side by a medial plate 90 downwardly received into a medial three-sided frame 92 of the localization framework 68. The breast is compressed from an outside (lateral) side of the breast by a lateral fence 94 downwardly received into a lateral three-sided frame 96 of the localization framework 68, defining an X-Y plane. The X-axis is vertical (sagittal) with respect to a standing patient and corresponds to a left to right axis as viewed by a clinician facing the externally exposed portion of the localization fixture 16.

Perpendicular to this X-Y plane extending toward the medial side of the breast is the Z-axis, which typically corresponds to the orientation and depth of insertion of a probe 98 of a disposable probe assembly 100 of the MRI biopsy device 14 or of a sleeve trocar 102 with inserted introducer obturator 104. For clarity, the term Z-axis may be used interchangeably with "axis of penetration", although the latter may or may not be orthogonal to the spatial coordinates used to locate an insertion point on the patient. Versions of the localization fixture 16 described herein allow a nonorthogonal axis of penetration to the X-Y axis to a lesion at a convenient or clinically beneficial angle. An origin of the spatial coordinates may be imaging the dents imparted to the tissue by the lateral fence 94. Alternatively, a disposable fiducial pointer 106 held by a fiducial holder 108 is filled with an MRI imagable material (e.g., KY jelly, saline, gadolinium) and sealed with a cap 110.

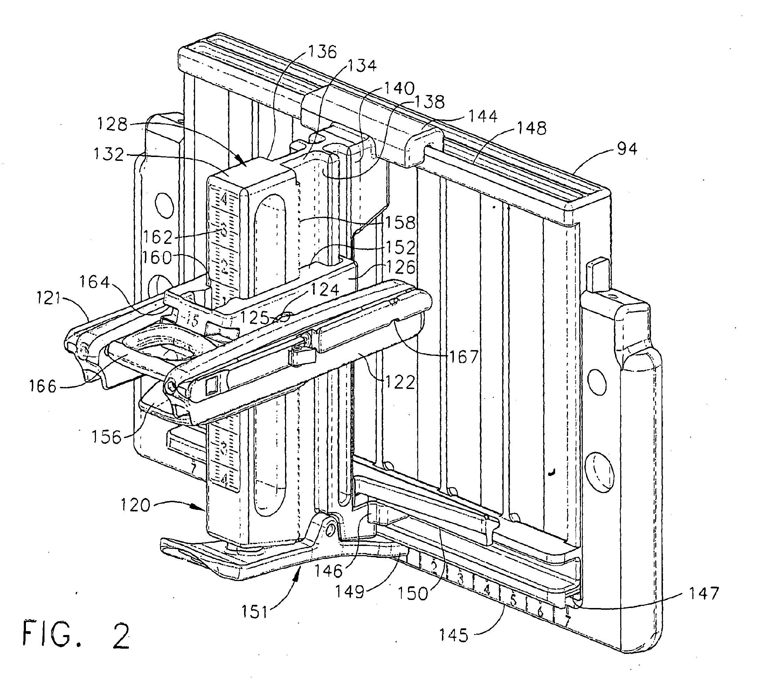

The probe 98, sleeve trocar 102 and fiducial pointer 106 are guided by the localization fixture 16. With particular reference to FIG. 2, a lateral fence supported pedestal 120 spatially positions left and right primary targeting rails 121, 122 that in turn guide the fiducial pointer 106, the sleeve / trocar 102, or the probe 98 of the biopsy device 14 (FIG. 1). The primary targeting rails 121, 122 each include an attachment axle 124 that receives in either a left or right side axle hub 125 of a (Y-axis) height yoke 126 that is vertically adjustable upon a pedestal main body 128, that in turn is laterally adjustable upon the lateral fence 94. Alternatively, a breast coil may enable mounting the pedestal main body on the medial plate 90 for accessing medially. The pedestal main body 128 includes a proximal upright rectangular column 132 with a thinner wall 134 projecting from its distal side that flares laterally outward (defining left and right vertical rectangular slots 136, 138) as part of a bracket 140 with top and bottom hanger arms 144, 146 that slide laterally respectively on a top track 148 and a proximally open lower track 150 formed in the lateral fence 94. A lateral (X-axis) adjustment lever 151 may be raised to lift its distal end 149 out of engagement with a bottom track 147 formed in the lateral fence 94 as the lateral adjustment lever 151 is repositioned to the left or right to a desired location with reference to a lateral measurement guide 145.

The height yoke 126 is a rectangular cuff interrupted in a mid-portion of a distal side to form locking left and right hands 152 respectively which ride vertically in the left and right vertical rectangular slots 136, 138. The locking left and right hands 152 have respective ridged proximal surfaces (not shown) that are selectively drawn proximally into locking engagement by a height locking lever 156 with a ridged surface 158 on a proximal side of each vertical rectangular slot 136, 138. Lifting the height locking lever 156 takes the height yoke 126 out of locking engagement to the pedestal main body 128 as the height yoke 126 is vertically repositioned. For height adjustment, the proximal top surface of the height yoke 126 serves as a sight 160 to read a height measurement scale 162 presented on a proximal surface of the height locking lever 156.

The attachment axle 124 allows rotation so that an axis of penetration may include an upward or downward trajectory. In the illustrative version, proximal corners of the height yoke 126 include angle detents 164 (e.g., -15°, 0°, +15°) that are selectable by an angle lock lever 166. The primary targeting rail 122 includes a distal detent 167 that serves as a home reference for the fiducial holder 108 (FIG. 1).

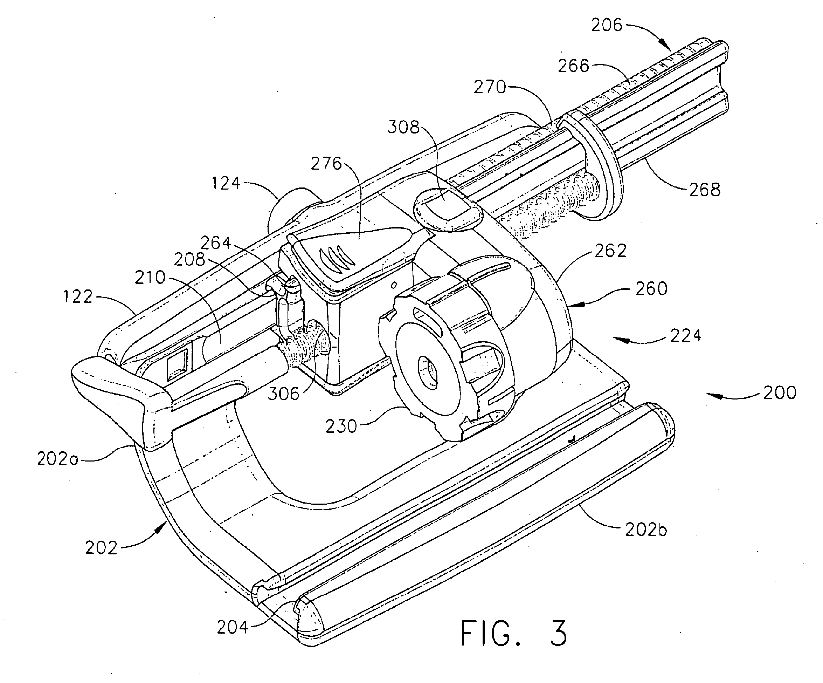

In FIGS. 3-4, a guidance assembly 200, that may be attached to the lateral fence supported pedestal 120 of FIG. 2, includes a cradle 202 whose upper lateral side 202a flares upwardly to engage a bottom channel 203 of the primary targeting rail 122. A lower lateral side 202b flares horizontally to provide a holster guide track 204 that underlies the axis of penetration. To provide additional guidance to the MRI biopsy device 14 (FIG. 1), a secondary targeting rail 206 includes a lateral channel 208 that is guided along a longitudinal guide tab 210 of the primary targeting rail 122. When fully engaged thereon, a pawl 212 pivoting under urging of a pawl spring 214 about a vertical pawl pin 216 in a lateral window 218 proximally positioned in the secondary targeting rail 206 drops into a proximal detent 220 proximally positioned on the primary targeting rail 122. The pawl spring 214 may maintain the pawl 212 in a neutral position that serves in both assembly and later removal of the secondary targeting rail 206 or comprises a pair of opposing pawl springs (not shown) for that purpose.

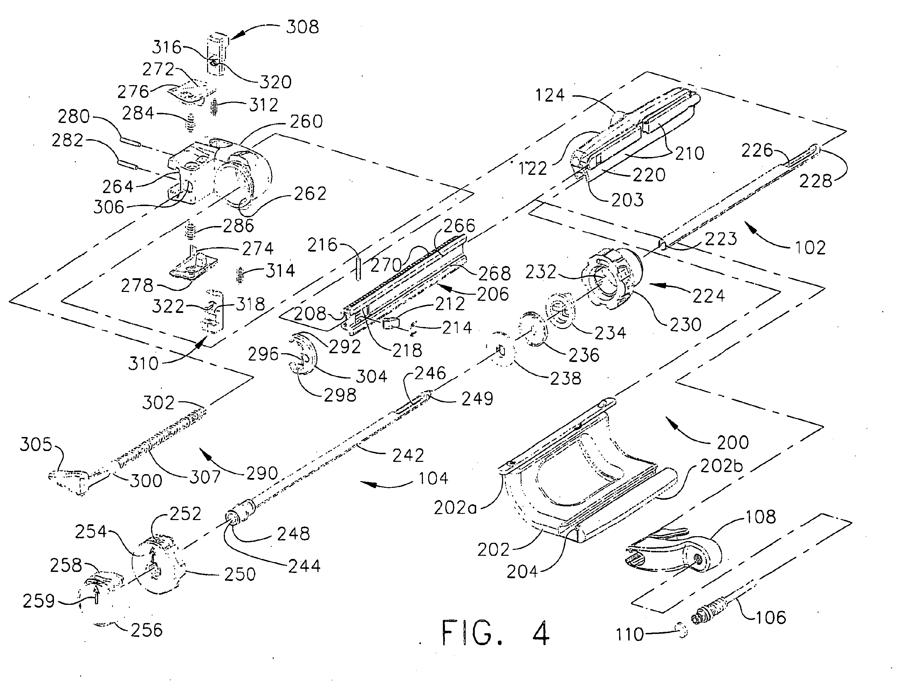

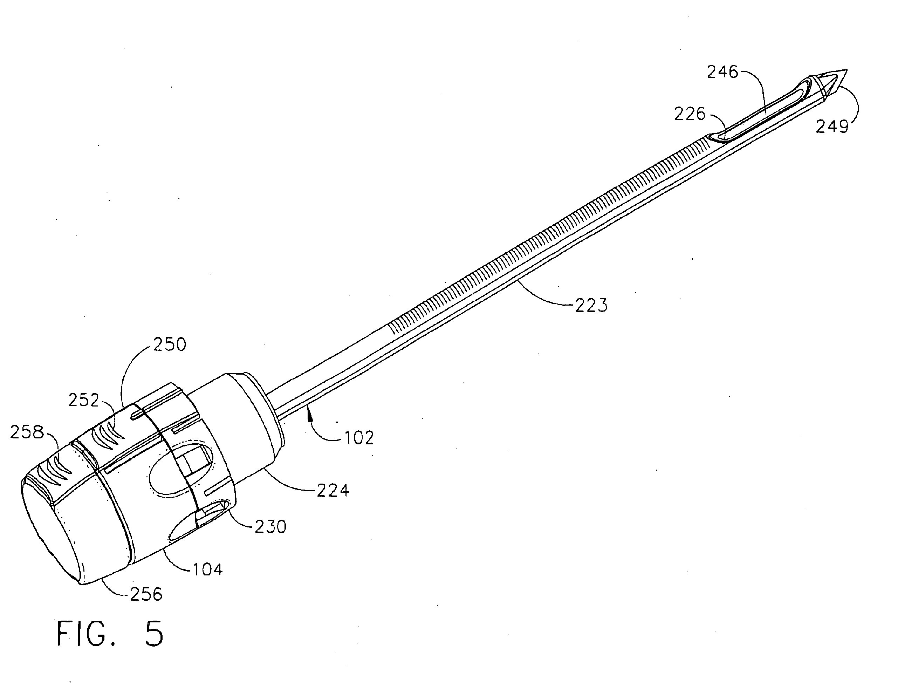

In FIGS. 4-5, the sleeve trocar 102 includes a hollow shaft (or cannula) 223 that is proximally attached to a cylindrical hub 224 and has a lateral aperture 226 proximate to an open distal end 228. The cylindrical hub 224 has an exteriorly presented thumbwheel 230 for rotating the lateral aperture 226. The cylindrical hub 224 has an interior recess 232 that encompasses a duckbill seal 234, wiper seal 236 and a seal retainer 238 to provide a fluid seal when the shaft 223 is empty and for sealing to the inserted introducer obturator 104 which is not in accordance with the invention.

The introducer obturator 104 advantageously incorporates a number of components with corresponding features. A hollow shaft 242 includes a fluid lumen 244 that communicates between an imageable side notch 246 and a proximal port 248. The hollow shaft 242 is longitudinally sized to extend when fully engaging a piercing tip 249 out of the distal end 228 of the sleeve trocar 102. An obturator handle 250 encompasses the proximal port 248 and includes a locking feature 252, which includes a visible angle indicator 254, that engages the sleeve thumbwheel 230 to ensure that the imageable side notch 246 is registered to the lateral aperture 226 in the sleeve trocar 102. An obturator seal cap 256 may be engaged proximally into the obturator handle 250 to close the fluid lumen 244. The obturator seal cap 256 includes a locking or locating feature 258 that includes a visible angle indicator 259 that corresponds with the visible angle indicator 254 on the obturator thumbwheel cap 230. The obturator seal cap 256 may be fashioned from either a rigid, soft, or elastomeric material.

Returning to FIGS. 3, 4, the sleeve trocar 102 is guided, during penetration of tissue, by a sleeve mount 260 having a sleeve hub 262 that receives the cylindrical hub 224 of the sleeve trocar 102. The sleeve mount 260 has a lateral sleeve hub channel 264 that slides along top and bottom guide flanges 266, 268 of the secondary targeting rail 206, each having an aligned and recess ridged, ratcheting surface 270 that interacts with a respective top and bottom ratcheting feature 272, 274 on respective top and bottom rail lock rocker latches 276, 278 that are engaged by respective top and bottom latch pins 280, 282 in respective sides of the sleeve mount 260. The ratcheting features 272, 274 are proximally ramped such as to allow distal movement. Distal portions of each rail lock rocker latches 276, 278 are biased away from the sleeve mount 260 by respective rail lock compression springs 284, 286 to bias the ratcheting features 272, 274 into contact with the ridges surfaces 270 of the guide flanges 266, 268. Simultaneous depression of the rail lock rocker latches 276, 278 allow the sleeve mount 260 to be drawn proximally, withdrawing any sleeve trocar 102 supported therein, until the sleeve mount 260 reaches a proximal end of the secondary targeting rail 206, whereupon the sleeve mount 260 rotates the pawl 212 clockwise (as viewed from the top) and is thus engaged to the secondary targeting rail 206 as the secondary targeting rail 206 is unlocked from the primary targeting rail 122, causing removal therefrom with continued proximal movement.

Before mounting the secondary targeting rail 206 onto the primary targeting rail 122 in the first place, the sleeve mount 260 is advantageously adjustably positioned on the secondary targeting rail 206 to set a desired depth of penetration. In particular, a depth guide 290 is formed by a crescent-shaped depth indicator 292 having a lateral channel 296 shaped to engage the top and bottom guide flanges 266, 268. Forward ramped surfaces 298 on the top and bottom of the lateral channel 296 are positioned to engage the ridged ratcheting surfaces 270 on the secondary targeting rail 206, allowing assembly by inserting the depth indicator 292 from a distal end of the secondary targeting rail 206. Frictional engagement thereafter resists further proximal movement and strongly opposes any distal movement, especially from a depth lead screw 300 of the depth guide 290, whose distal end 302 rotates within an outboard hole 304 in the depth indicator 292 and whose proximal end deflects laterally as a depth actuator lever 305 is used to rotate and longitudinally position the depth lead screw 300 therein. A mid portion of the depth lead screw 300 is received in a longitudinal through hole 306 formed in the sleeve mount 260 outboard of its lateral channel 208. For coarse depth adjustment, outer lead threads 307 on the depth lead screw 300 selectively engage the sleeve mount 260 until top and bottom coarse adjust buttons 308, 310 are inwardly depressed into the sleeve mount 260, compressing respective top and bottom coarse adjust compression springs 312, 314. Each coarse adjust button 308, 310 includes a respective vertically elongate aperture 316, 318 whose inward surface presents a worm gear segment 320, 322 to engage the outer lead threads 307 on the depth lead screw 300 when urged into engagement by relaxed coarse adjust compression screws 312, 314.

Returning to FIG. 3, the thumbwheel 230 is depicted as engaged to the sleeve hug 262 of the sleeve mount 260 with other portions of the sleeve trocar 102 omitted. Application s consistent with the present invention may include a probe of an MRI biopsy device that includes a piercing tip or that otherwise is used without passing through a hollow shaft (cannula) 223. As such, the thumbwheel with similar sealing members may be incorporated into the sleeve mount 260.

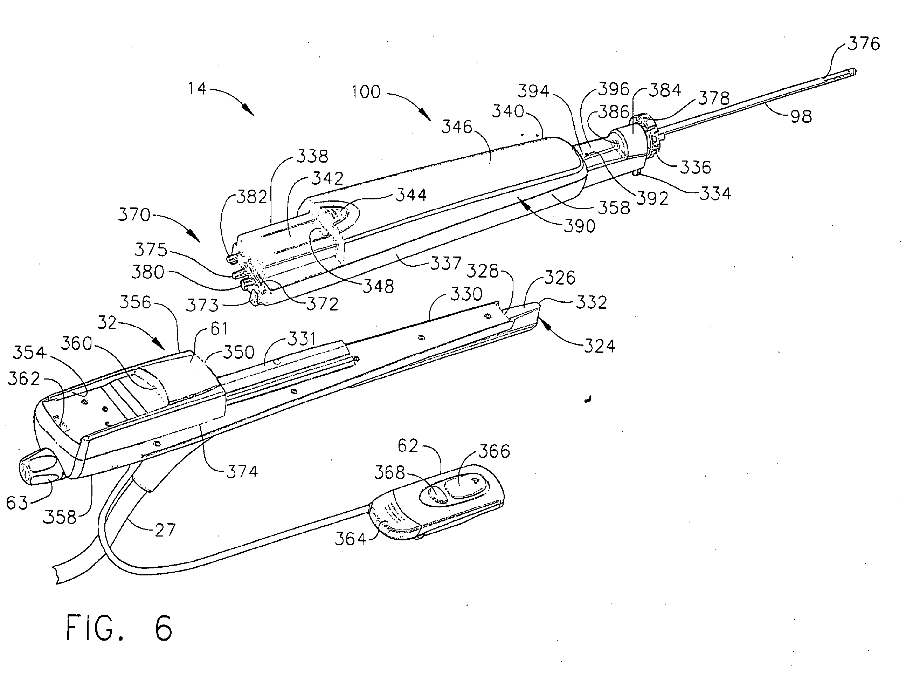

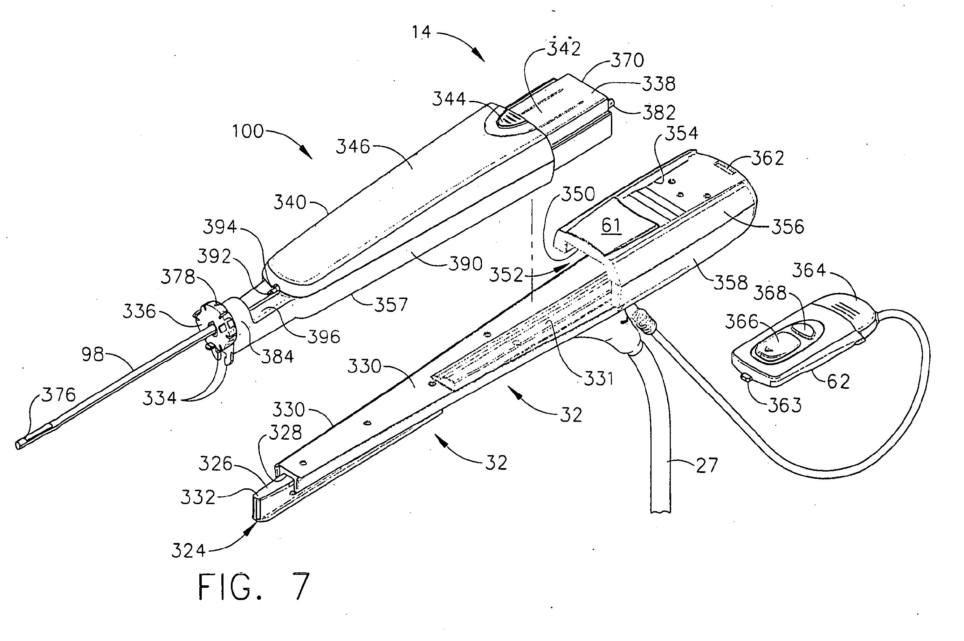

In FIGS. 6-7, the MRI biopsy device 14 has the disposable probe assembly 100 depicted detached from the reusable holster portion 32 and with the remote keypad 62 released from the reusable holster portion 32. The sheathed cable 27 is joined to an underside of the reusable holster portion 32 distal to the aft end thumbwheel 63 to enhance balance and support of the reusable holster portion, which in turn may be engaged to the holster guide track 204 (FIG. 4) by an I-beam shaped holster rail 324 whose upper surface 326 is engaged within a bottom channel 328 of a holster base plate 330. A ridged member 331 upon the holster base plate 330 guides the disposable probe assembly 100 during engagement. A narrowed upper distal surface 332 of the holster rail 324 also engages downward gripping flanges 334 extending downward just proximal to a distal thumbwheel 336 of the disposable probe assembly 100. An under slung shell 337 is fastened to the proximal undersurface portion of the holster base plate 330.

The disposable probe assembly 100 also has an undersurface that backwardly slides into engagement with the reusable holster portion 32. In particular, a narrowed proximal end 338 is formed into an upper cover 340 with a distal locking arm 342 separated from the upper cover 340 on each side except proximally to present an unlocking button 344 on an exposed surface 346 of the upper cover 340 that is depressed to disengage a locking surface 348 (FIG. 6) from a distal lip 350 of a distally open receiving aperture 352 in the reusable holster portion 32 of the holster plate 330.

A recessed deck 354 in an upper proximal surface of a proximal top cover 356 of the reusable holster portion 32 is shaped to receive the remote keypad 62. A lower shell 358 mates to the proximal top cover 356. The proximal top cover 356 also defines the upper portion of the receiving aperture 352. The recessed deck 354 has a front guide hole 360 and a back locking aperture 362 registered to respectively receive a front tooth 363 and a flexing unlock tab 364 at an aft end of the remote keypad 62 to selectively engage and disengage the keypad 62 from the reusable holster portion 32. The keypad 62 also includes a translation rocker button 366 that has a distal advance, a default neutral, and an aft retract command position. An aft button 368 may be programmed for mode functions such as saline flush.

With particular reference to FIG. 6, the disposable probe assembly 100 has a plurality of interconnections presented on an aft docking end 370. A rightward canted vacuum hose nib 372 is positioned to receive a vacuum conduit (not shown) that would be gripped by a friction clip 373 extending under and aft thereof to prevent inadvertent release. A right side slot 374 is distally open and formed between the holster base plate 330 and proximal top cover 356 to receive such a vacuum conduit as the disposable probe assembly 100 is engaged to the reusable holster portion 32. A center splined driveshaft 375 engages the aft end thumbwheel 63 and communicates with the distal thumbwheel 336 to rotate a side aperture 376 in probe 98 to a desired side, as visually confirmed by an arrow indicator 378 on the distal thumbwheel 336. A right splined driveshaft 380 effects cutter translation and a left splined driveshaft 382 effects cutter rotation.

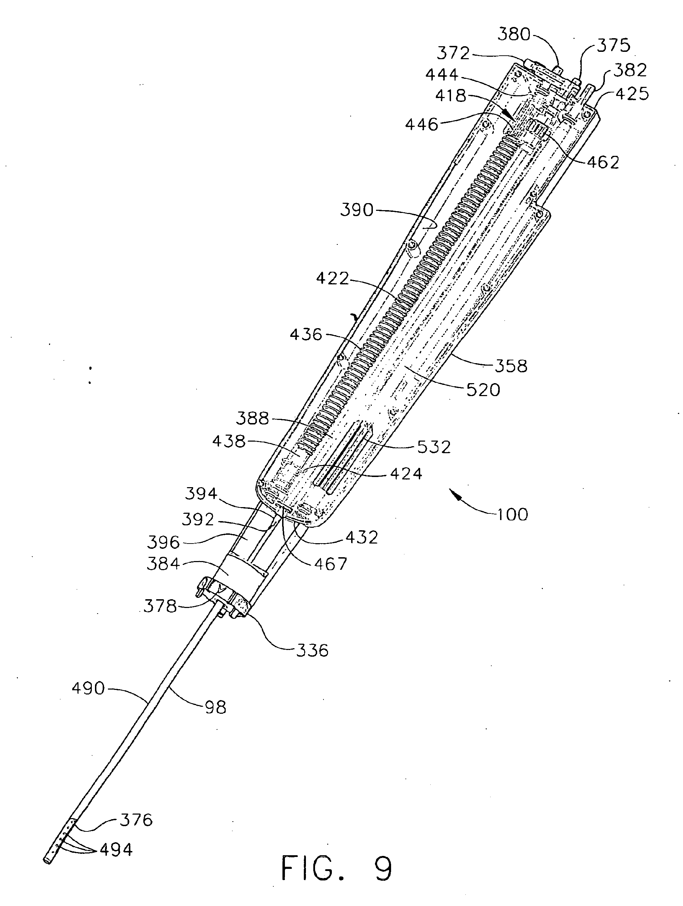

The distal thumbwheel 336 and probe 98 are mounted to a cylindrical hub 384, which is a distal portion of the lower shell 358 that extends beyond the mating with the upper cover 340. A sample through hole 386 communicates through the cylindrical hub 384 for receiving a rotating and translating cutter tube 388 (FIG. 9) that enters the probe 98 and for receiving tissue samples (not shown) deposited by a retracting cutter tube 388. As the cutter tube 388 fully retracts into a carriage cavity 390 formed between the upper cover 340 and proximal portion of the lower shell 358, a distally extending tip 392 from a vacuum tube 394 encompassed by the cutter tube 388 dislodges the retracted tissue sample onto a sample retrieval platform 396, which is a relieved area between the upper cover 340 and the cylindrical hub 384.

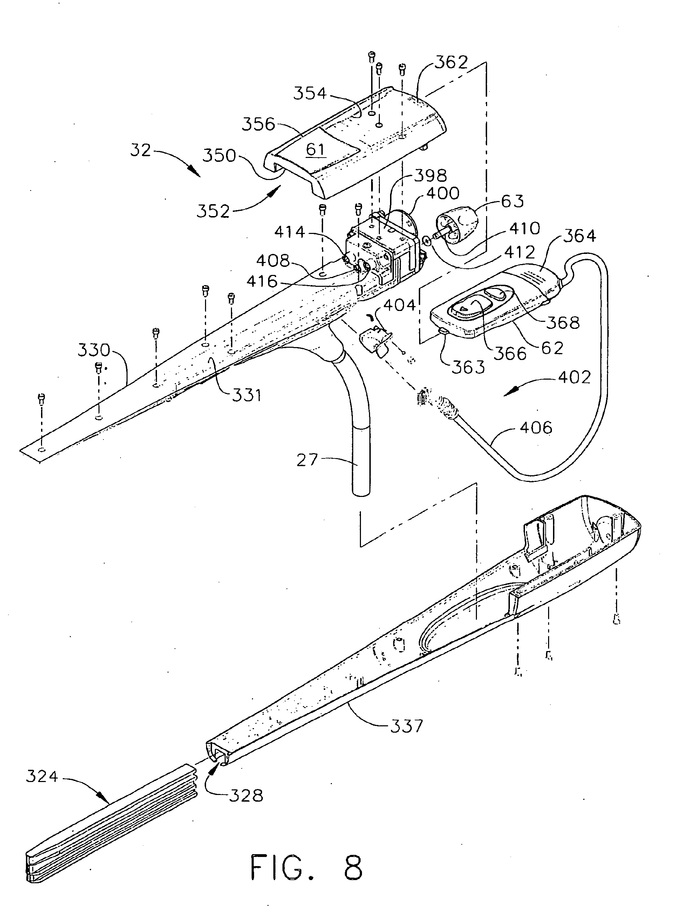

In FIG. 8, it should be appreciated that the sheathed cable 27 connects to the holster base plate 330 and communicates a single mechanical drive rotation to a fixed ratio transmission 398 mounted to the holster base plate 330 and electrically communicates with an encoder 400 coupled to the fixed ratio transmission 398 aft of the receiving aperture 352. The sheathed cable 27 also communicates electrically with the display area 61 via a wire bundle (not shown) and with the keypad 62 via a cable assembly 402, the latter including a strain relief bracket 404 that grips a keypad cable 406 and is fastened proximate to the sheathed cable 27. The fixed ratio transmission 398 has a pass-through port 408 that receives a distal end the center splined driveshaft 375 (FIG. 6) to rotatingly engage a proximally received beveled shaft 410 distally presented by the aft end thumbwheel 63 and sealed by an O-ring 412. A right port 414 distally presented by the fixed ratio transmission 398 engages for rotation the right splined driveshaft 380 from the disposable probe assembly 100 for advancing and retracting ("translation") the cutter tube 388. A left port 416 distally presented by the fixed ratio transmission 398 engages for rotation the left splined driveshaft 382 from the disposable probe assembly 100 for rotating the cutter tube 388 when a distal cutting edge of the cutter tube 388 slides past the side aperture 376 of the probe 98.

In FIGS. 9-10, the carriage cavity 390 of the disposable probe assembly 100 includes a cutter carriage 418 having a threaded longitudinal bore 420 that encompasses an elongate translation shaft 422 whose proximal termination is the right splined driveshaft 380 supported by an aft right cylindrical bearing 424 received in an aft wall 425 of the lower shell 358. A race about the outer circumference of the cylindrical bearing 424 receives an O-ring 426. A distal end 428 of the threaded translation shaft 422 rotates within a distal right cylindrical bearing 430 engaged to a forward wall 432 of the lower shell 358. A race about the outer circumference of the cylindrical bearing 430 receives an O-ring 434. A threaded central portion 436 of the elongate translation shaft 422 resides between an unthreaded distal over-run portion 438 and an unthreaded proximal over-run portion 440, both sized to allow the threaded longitudinal bore 420 of the cutter carriage 418 to disengage from the threaded central portion 436.

A distal compression spring 442 and a proximal compression spring 444 respectively reside on the unthreaded distal and proximal over-run portions 438, 440 to urge the threaded longitudinal bore 420 of the cutter carriage 418 back into engagement with the threaded central portion 436 upon reversal of rotation of the elongate translation shaft 422. In particular, the cutter carriage 418 includes a top longitudinal channel 446 that slidingly engages an undersurface of the upper cover 340 (not shown) and a bottom longitudinal guide 448 that engages a longitudinal track 450 on a top surface of the lower shell 358. Thus rotationally constrained, rotation of the elongate translation shaft 422 causes corresponding longitudinal translation of the cutter carriage 418 with distal and aft pairs of gripping flanges 452, 454 maintained laterally to the left to engage respectively distal and proximal races 456, 458 formed on each side of a toothed portion 460 of a cutter spur gear 462, which has a longitudinal bore for applying vacuum.

To that end, the vacuum hose nib 372 is attached to a mounting structure 464 that is gripped between the upper cover 340 and the lower shell 358 to present an orifice 466 within the carriage cavity 390 that is aligned with the longitudinal bore of the cutter gear 462 and that is in fluid communication with the vacuum hose nib 372.

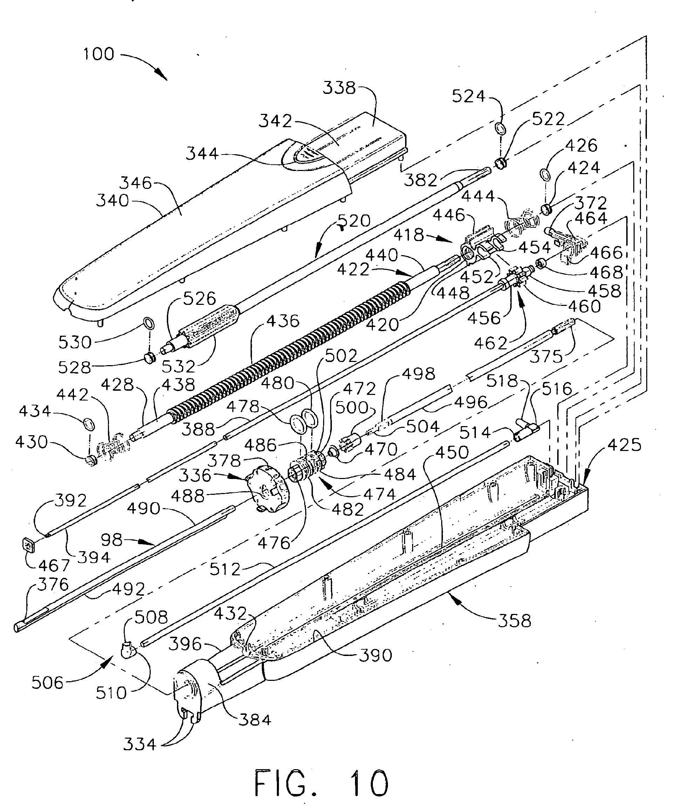

With particular reference to FIG. 10, the proximal end of the vacuum tube 394 is received in the orifice 466. A rectangular guide 467 supports the distally extending tip 392 of the vacuum tube 394 and is engaged between the upper cover 340 and the lower shell 358. The cutter tube 388 encompasses and translates relative to the vacuum tube 394. A seal cap 468 attached to a proximal end of the cutter gear 462 dynamically seals to the outer circumference of the vacuum tube 394 so that vacuum pressure supplied proximate to the distally extending tip 392 is not released within the carriage cavity 390. The cutter tube 388 is advanced around the open distal end of the vacuum tube 394, across the sample retrieval platform 396 to seal against a back seal 470 that substantially closes a proximal opening 472 into a sleeve union 474 that rotates within the cylindrical hub 384. The sleeve union 474 has a distal end 476 engaged for rotation with the distal thumbwheel 336. Distal and proximal O-rings 478, 480 reside respectively within distal and proximal races 482, 484 that straddle a lateral passage 486 of the sleeve union 474 to provide a degree of frictional resistance against inadvertent rotation and advantageously seal the lateral passage 486 for vacuum assistance to prolapse tissue and to retract samples. A noncircular opening 488 is centered in a distal face of the distal thumbwheel 336. A proximal end of a probe tube 490 of the probe 98 extends through the noncircular opening 488 to receive a distal end of the cutter tube 388. A lateral tube 492 attached along its length to the probe tube 490 communicates with the lateral passage 486 of the union sleeve 474. The lateral tube 492 defines a lateral lumen that communicates with the a cutter lumen defined by the probe tube 490 / cutter tube 388 below the side aperture 376 through lumen holes 494 (FIG. 9).

The center splined driveshaft 375 that is turned by the aft end thumbwheel 63 rotates in turn a shaft 496 whose keyed distal end 498 in turn is engaged to and rotates a pinion gear 500 that is in gear engagement to a proximal spur gear 502 that forms an outer proximal circumference of the sleeve union 474. A cylindrical distal tip 504 of the keyed distal end 498 rotates within an axle hole (not shown) in the lower shell 358. Rotation of the aft end thumbwheel 63 thus rotates the probe 98.

A distal elbow pneumatic fitting 506 is supported in the lower shell 358 to have an upper end 508 communicating with the lateral passage 486 of the sleeve union 474 and an aft end 510 attached to a vent pneumatic conduit 512 supported by the lower shell 358. The other end of the vent pneumatic conduit 512 is attached to a distal end 514 of a proximal elbow pneumatic fitting 516 whose lateral end 518 is open to atmosphere. Sizing of various components that vent atmospheric pressure through the lumen holes 494 from the lateral end 518 are such that a tissue sample may be withdrawn through the probe tube 490. Yet a greater pneumatic draw of air through the vacuum hose nib 372 prior to severing a tissue sample results in a sufficient low pressure at the side aperture 376 to prolapse tissue for severing.

An elongate rotation shaft 520 proximally terminates in the left splined driveshaft 382 that is supported for rotation by a left aft cylindrical bearing 522 having a race about an outer circumference that receives an O-ring 524 and is received in the aft wall 425 of the lower shell 358. A distal end 526 of the elongate rotation shaft 520 is received for rotation in a left distal cylindrical bearing 528 having a race about an outer circumference that receives an O-ring 530 and that is received within the front wall 425 of the lower shell 358. As the cutter carriage 418 advances to position the cutter tube 388 to slide past the side aperture 376, the cutter spur gear 460 engages a spur gear portion 532 of the elongate rotation shaft 520. Rotating the cutter tube 388 in proportion to an amount of rotation advantages secures an effective severing of tissue. Eliminating rotation when not severing advantageously enhances retraction of tissue sample retraction.

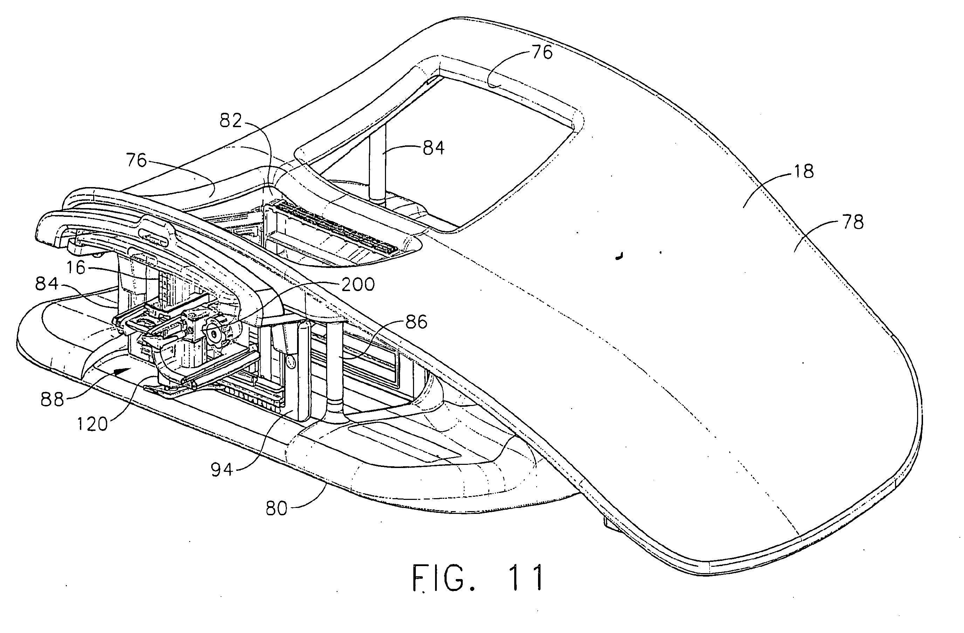

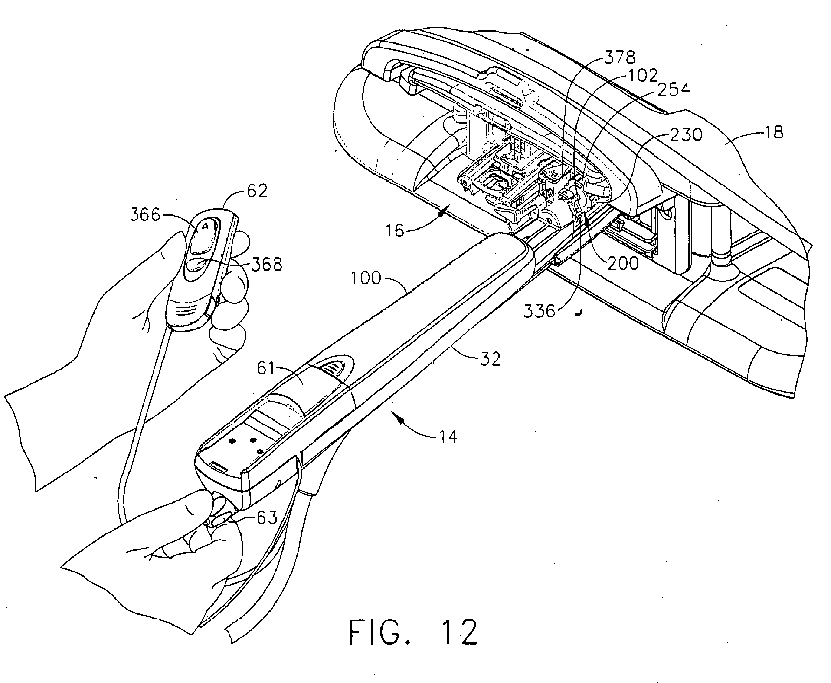

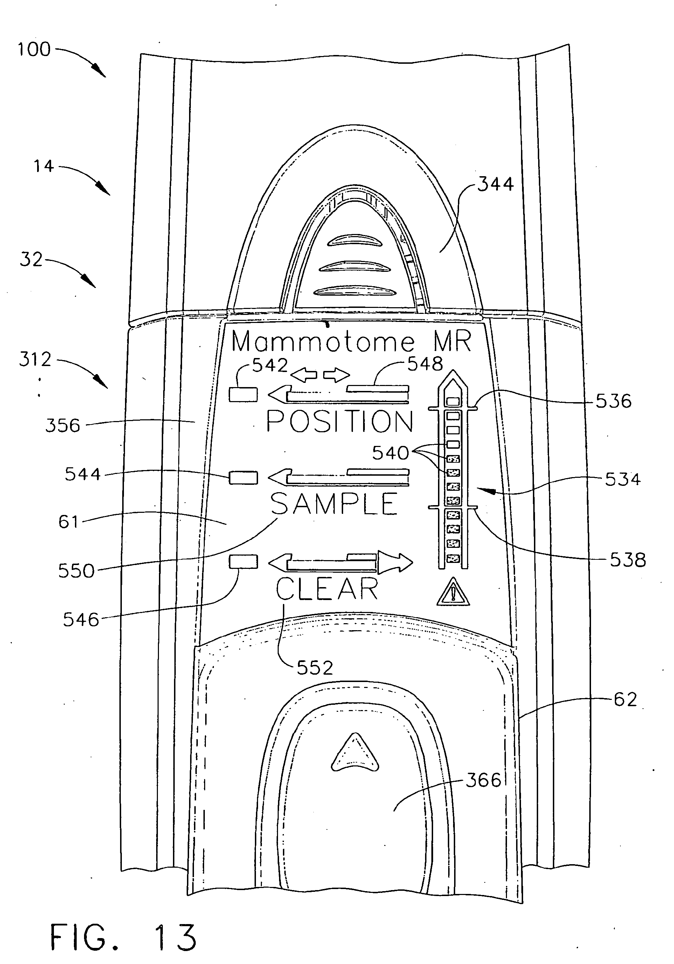

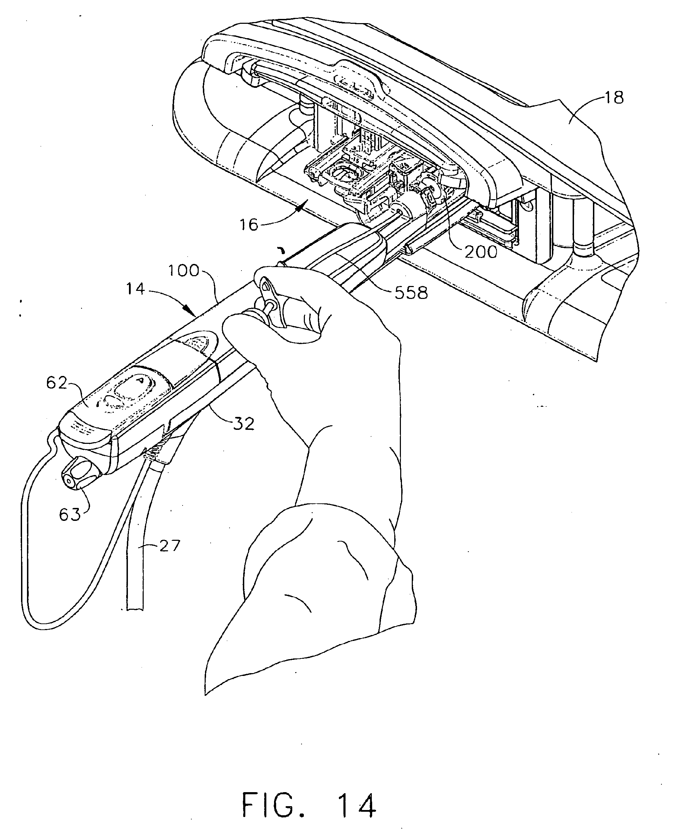

In use, in FIG. 11, the localization fixture 16 has been installed into the breast coil 18. The guidance assembly 200 has been preset for a desired insertion point, a desired axis of penetration, and a depth of penetration. After the sleeve trocar 102 / introducer obturator 104 have been inserted and imaged to confirm placement, the introducer obturator 104 is removed and the probe 98 of the biopsy device 14 is inserted, as depicted in FIG. 12. The shape of the sleeve trocar 102 aligns the probe 98, visually assisted by lining up the arrow indicator 378 on the distal thumbwheel 336 with the visible angle indicator on the thumbwheel 230 of the sleeve trocar 102. The surgeon may effect operation of the biopsy device 14 by depressing the translation rocker button 366 and aft button 368 on the keypad 62 while referencing status information about the biopsy device 14 on the display area 61. In FIG. 13, the display area 61 advantageously includes a cutter position bar graph 534 having distal and proximal indications 536, 538 that may be compared with how many light segments 540 have been illuminated to indicate progress of the cutter tube 388 relative to the side aperture 376. The aft button 368 may be toggled to cycle the biopsy device 14 through three modes, indicated by a position LED indicator 542, a sample LED indicator 544, and a clear LED indicator 546 with a corresponding label that graphically depicts operation of the biopsy device in that mode. In particular, a position mode depiction 548 illustrates that the cutter tube 388 may be advanced and retracted, for instance, closing the side aperture 376 prior to insertion of the probe 98 into the sleeve trocar 102. In a sample mode depiction 550, vacuum assistance is implemented, drawing sufficient air through the cutter tube 388 to prolapse tissue into the open side aperture 376 that is maintained while translating the cutter tube 388. In a clear mode depiction 552, vacuum is maintained while fully retracting the cutter tube 388 to retract a tissue sample. In FIG. 14, a marker device 548 is deployed through the sample through hole 386 in the cylindrical hub 388.

### Biopsy Device With Energy Based Tissue Penetration

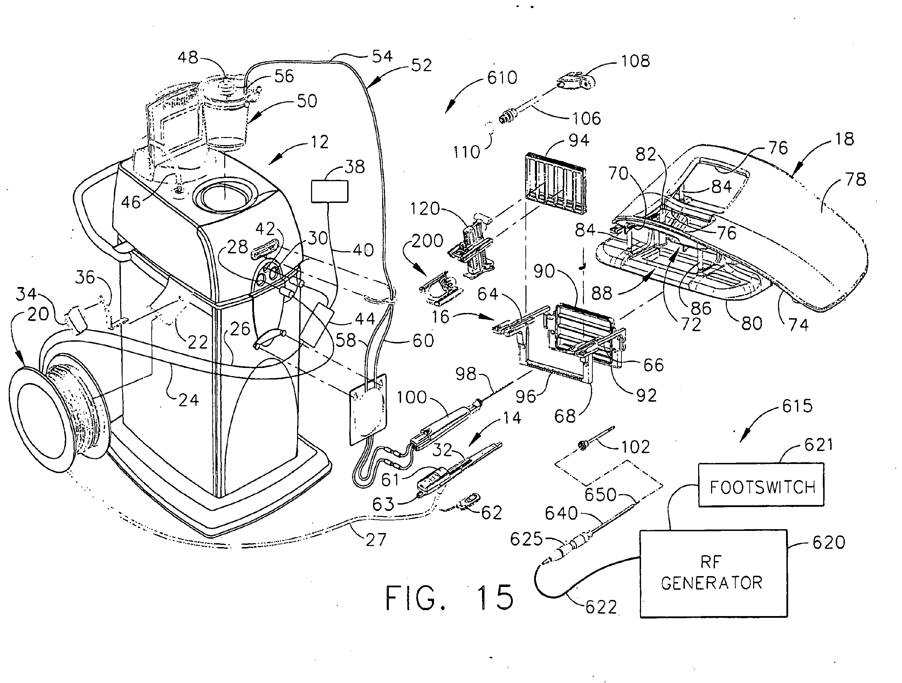

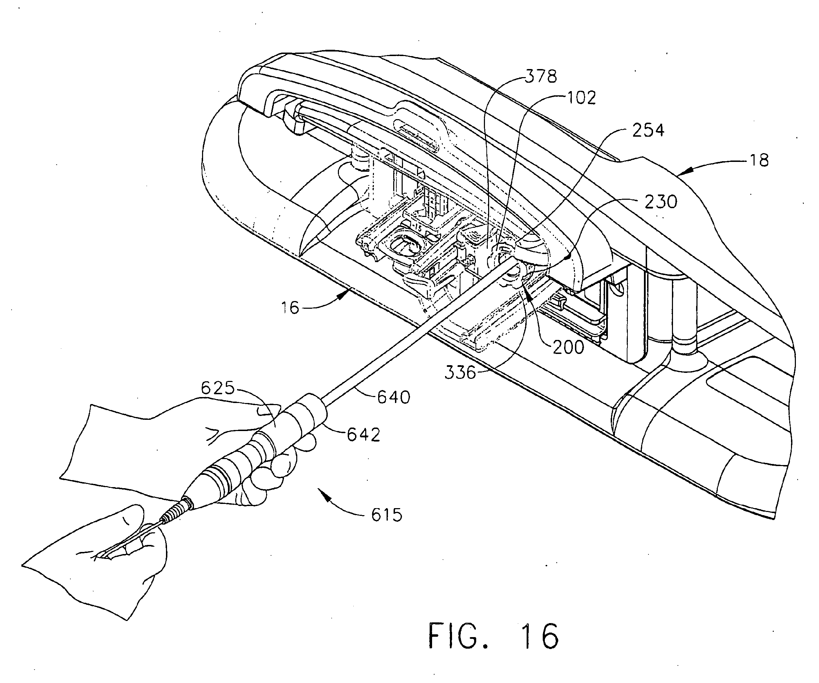

In FIG. 15, a surgical biopsy system 610 having an energy based tissue penetration system is shown. An energy based system can reduce force needed to penetrate hard tumors, improve hemostasis, and can adventageously move the tissue receiving lateral aperature 226 of the outer cannula or sleeve trocar 102 closer to a distal end of the sleeve trocar 102. Additionally, an energy based system can be used to ablate and cauterize tissue not accessable through the lateral aperature 226. As illustrated, an energy based system such as but not restricted to an ultrasonic penetration system 615 can be provided that includes a generator 620, that can be activated by switch 621, and a handpiece assembly 625 that is operably connected to generator 620 by cable 622. The sleeve trocar 102 can be a fixedly attached element of handpiece assembly 625 or, if desired can be removeably attached thereto. FIG. 16 shows the ultrasonic penetration system 615 inserted into the surgical system 610 to penetrate tissue.

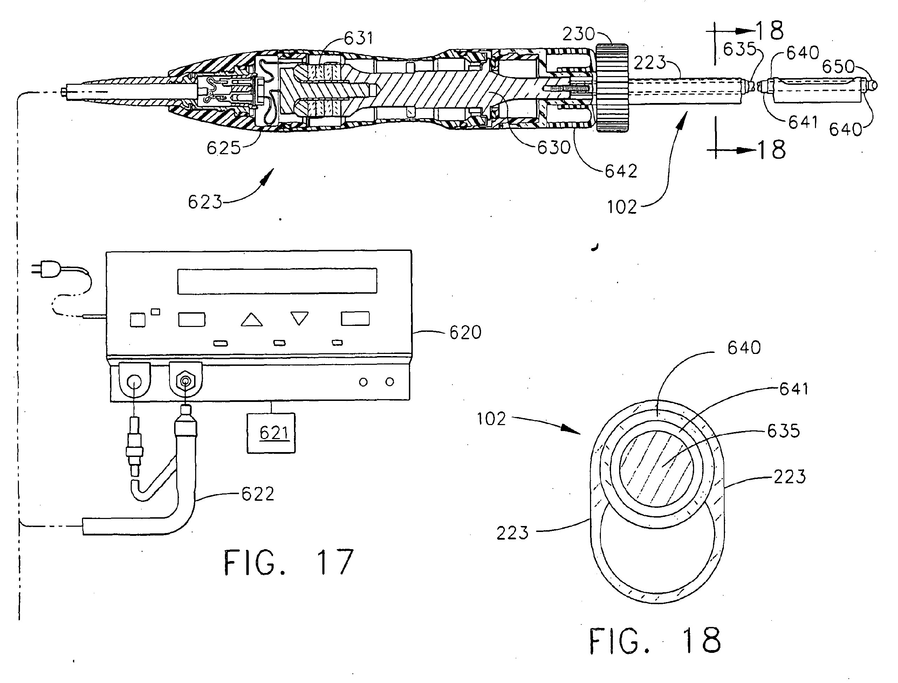

Turning now to FIG. 17, the generator 620 sends an electrical signal through a cable 622 at a selected amplitude, frequency, and phase to handpiece assembly 625. The handle assembly 625 includes an acoustic assembly 630 having one or more piezoelectric elements 631 capable of responding to electrical signals. Piezoelectric elements 631 expand and contract in response to the electrical signals, thereby converting the electrical energy into mechanical motion. The mechanical motion result in longitudinal waves of ultrasonic energy that propagate through the acoustic assembly 630 in an acoustic standing wave to vibrate the acoustic assembly 630 at a selected frequency, amplitude and phase, any of which may be a function of time or other variables. A blade or vibrating member 635 is removeably attached to accoustic assembly 630. A handle assembly 625 includes the proximal portion of acoustic assembly 630 up to the detachable vibrating member 635 extending distally therefrom. A handle 626 surrounds the proximal portion of acoustic assembly 630 and prevents surgeon contact with vibrating elements. A sleeve 640 can be removeably attached to the handle 626 with a cap 642 and surround the vibrating member 635 leaving a distal tip 650 exposed. Isolators 641 vibrationally isolate vibrating member 635 from stationary sleeve 640.

The parts of the acoustic assembly 630 can oscillate or resonate at the same resonant frequency. The elements contained therein are tuned so as to amplify motion of distal tip 650 and can provide harmonic vibration in resonance with the rest of the acoustic system, which produces the maximum back and forth motion of the distal tip 650. Distal tip 650 of acoustic assembly 630 can be placed in contact with tissue of the patient to transfer the ultrasonic energy to the tissue.

As the distal tip 650 vibrationaly couples with the tissue, cavitation, cell disruption, emulsification of tissue can occur. Thermal energy or heat can be generated with the side of the vibrating tip 650 producing cauterization and increased hemostasis as a result of internal cellular friction within the tissue. The heat produced may be sufficient to break protein hydrogen bonds, causing the highly structured protein (i.e., collagen and muscle protein) to denature (i.e., become less organized). The amount of cutting as well as the degree of coagulation obtained can vary with the vibrational amplitude of the distal tip 650, the amount of pressure applied by the user, and the sharpness of the distal tip 650. The distal tip 650 of the acoustic assembly 630 may focus the vibrational energy onto tissue directly in contact with the distal tip 650, and can intensify and localize thermal and mechanical energy delivery. A sleeve trocar 102 is shown removeably attached to handpiece assembly 623. Cross sectional FIGURE 18 is taken along lines A-A and shows the assembly of the vibrating member 635, the sleeve 640, and a hollow shaft or cannula 223. Sleeve 640 can block the aperture 226 in cannula 223 to prevent tissue contact with vibrating member 625.

This is merely a general overview of the operation of the ultrasonic system 615 and one of skill in the art will appreciate how the specific components operate to accomplish the energy based surgical action. It will further be understood that the ultrasonic device set forth in FIGS. 15 and 16 and the above-described elements above is merely exemplary such as those disclosed in U.S. Pat.. No, 6,274,963 by B. Estabrook et al. entitled "Methods and Devices for Controlling the Vibration of Ultrasonic Components".

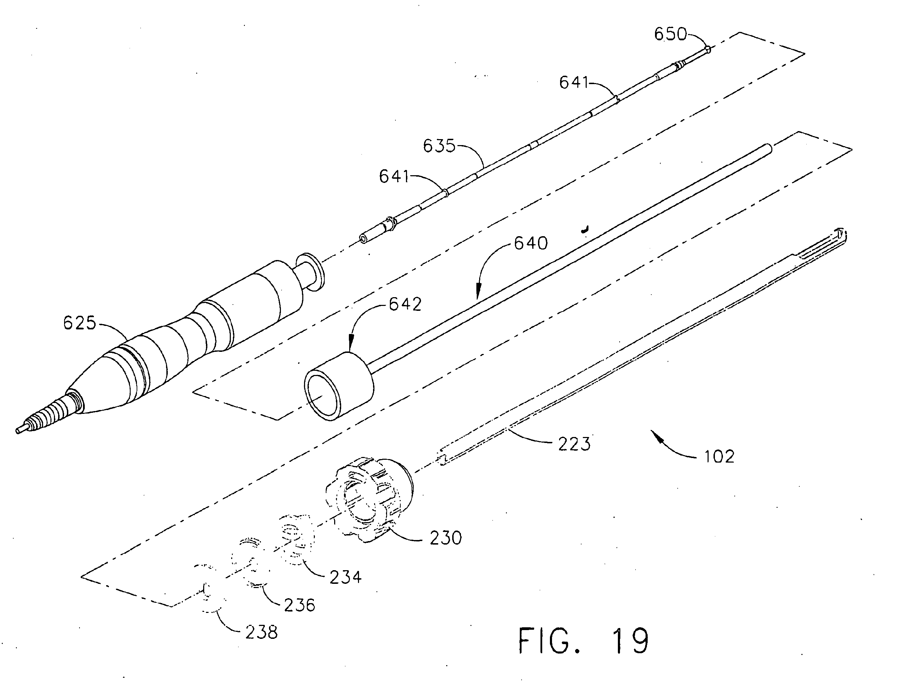

Figure 19 shows an exploded view of the elements of the handpiece assembly 623 and sleeve trocar 102. Vibrating member 635 removeably attaches to to accoustic assembly 630 in handle 625, and sleeve 640 slides over vibrating member 635 to removeably attach to handle 625 with collar 642. Handpiece assembly 623 slidingly and removeably mounts within sleeve trocar 102.

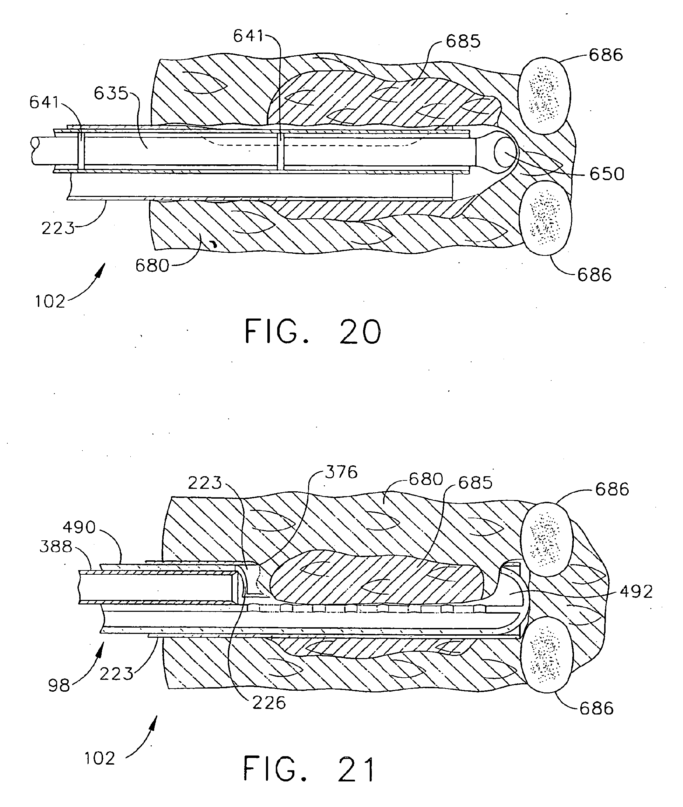

FIGS. 20-23 show how the ultrasonic penetration system 615 can offer advantages during biopsy surgery. Turning now to FIG. 20, a cross sectional view of the distal end 650 of handpiece 623 of the ultrasonic penetration system 615 is shown tunneling through a breast 680 and through a hard tumor 685. Ribs 686 are shown adjacent to the tumor 685, and rather than pushing the hard tumor 685 to one side, the vibrating tip 650 has tunneled through the hard tumor 685 and tissue adjacent to the ribs 686.

In FIG. 21, the handpiece assembly 623 has been withdrawn from the sleeve trocar 102. With the sleeve trocar 102 still in place in the breast, the probe 98 of the MRI biopsy device handpiece 14 (FIG. 1) has been placed in hollow shaft or cannula 223. A tunnel drilled through the breast 80 and tumor 85 with the distal tip 650 enables the hollow shaft or cannula 223 of sleeve trocar 102 to be pushed closer to the ribs 686. The movement of sleeve trocar 102 closer to the ribs provides the surgeon with access to a portion of tumor 685 close to the ribs 686. When probe 98 of the MRI biopsy device handpiece 14 is in position under lateral aperture 226, vacuum is used to draw tissue from the hard tumor 685 into lateral aperture 226 of sleeve trocar 102 and into the side aperture 376 of probe 98.

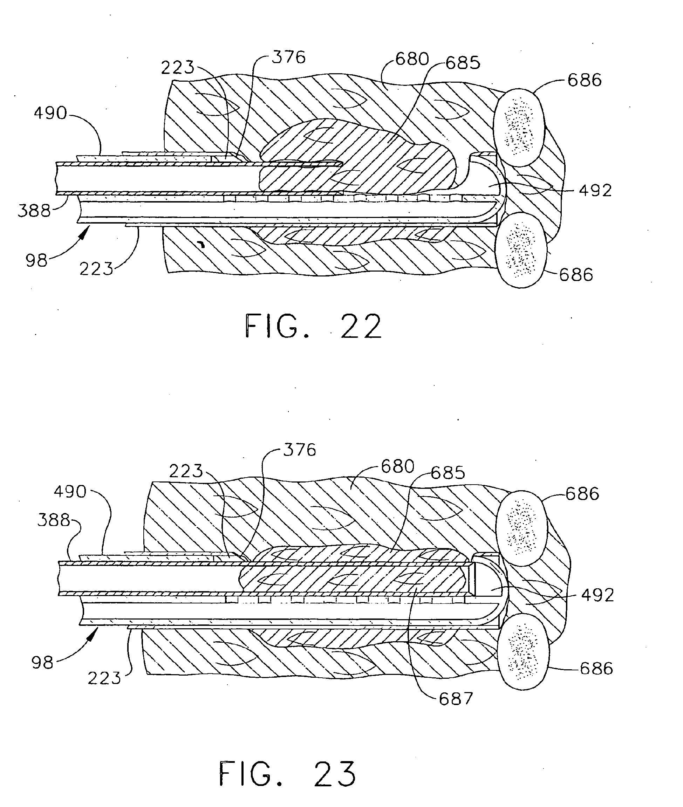

In FIG. 22, the rotating and translating cutter tube 388 is translating distally within lateral tube 492 of probe 98 and has partially severed the hard tumor 685. In FIG. 23, a first portion 687 of the hard tumor 685 is fully severed within the rotating and translating cutter tube 388.

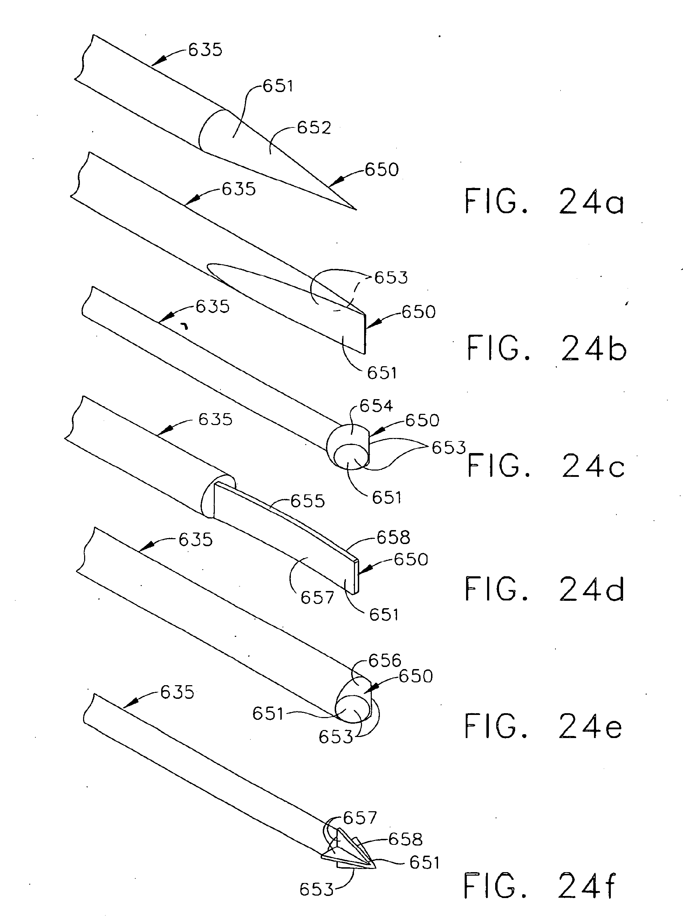

Figure 24 shows a number of alternate tip embodiments for the distal tip 650 of the vibrating member 635. Each distal tip 650 has at least one active surface 651 exposed to penetrate, cut, or coagulate tissue. Each of the at least one active surfaces 651 of the present invention can be conical, angular, spherical, concave, convex, arcuate, semi-spherical, sharp, or any combination thereof. FIG. 24a has a conical surface 652, and FIG. 24b has at least one angled surface 653. FIG. 24c shows a ball surface 654 combined with at least one angled surface 653, and FIG. 24d shows a distal tip 650 with at least one arcuate surface 655 and at least one concave surface 657 and a convex surface 658. FIG. 24e shows a distal tip 650 having a semi-spherical surface 656 and at least one angled surface 653. Figure 24f shows a distal tip 650 having at least one angled surface 653 and at least one concave surface 657. Additionally, any of the edges above can be a cutting sharp 659. Whereas the above described distal tips 650 can be used with the present device, the above list of surfaces is not meant to limit in any way the number of distal tips 650 that can function within the scope of the present invention.

### Handheld Biopsy System With Energy Tissue Penetration

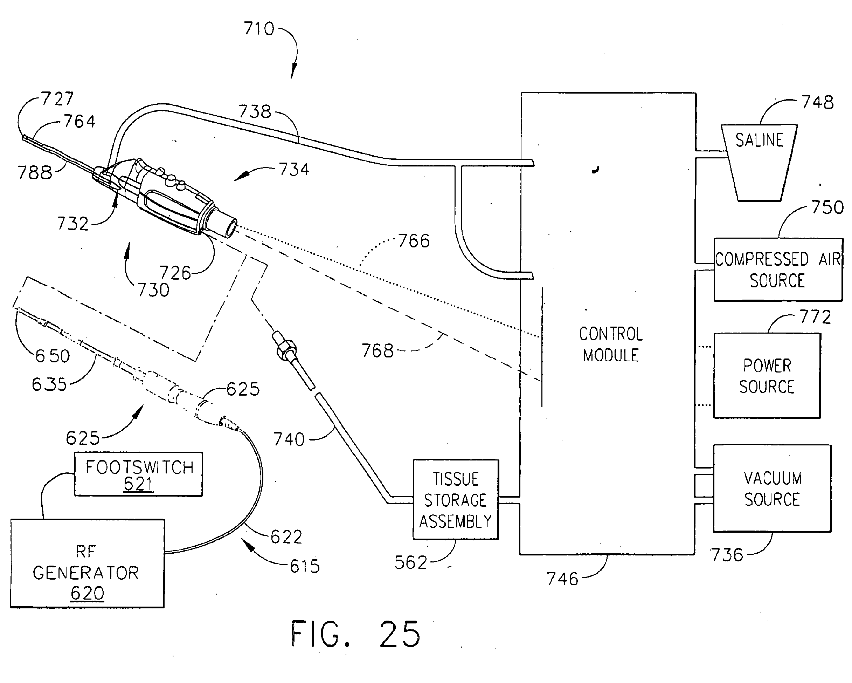

Whereas the above embodiment of the present invention combined the elements of surgical system 10 shown in FIG. 1 with an ultrasonic penetration system 615, an alternate embodiment of the present invention can be created by combining the an ultrasonic penetration system 615 with a handheld biopsy device. FIGS. 25 -28 show a complete handheld biopsy system 710 that can be combined with the ultrasonic penetration system 615. Handheld biopsy system 710 includes a handpiece assembly 730 comprising a handle 732 and a detachably connected holster 734. In FIG. 25, the handpiece assembly 732 has a distal biopsy needle probe 788 with side aperture 764 extending therefrom. Holster 734 can contain switches and controls. A control cord 766 and a rotating shaft 768 connect detachably connected holster 734 to a control module 746 and to biopsy needle probe 788. Control module 746 provide proximal probe assembly 732 with rotational motion to power handpiece assembly 730, and a microprocessor control of saline 748, compressed air 750, electrical power 772 and vacuum delivery 736. A first lateral tube 738 extends from probe assembly 732 to control module 746 to connect a vacuum source 736, saline source 756, and compressed air source 750 to handpiece assembly 730.

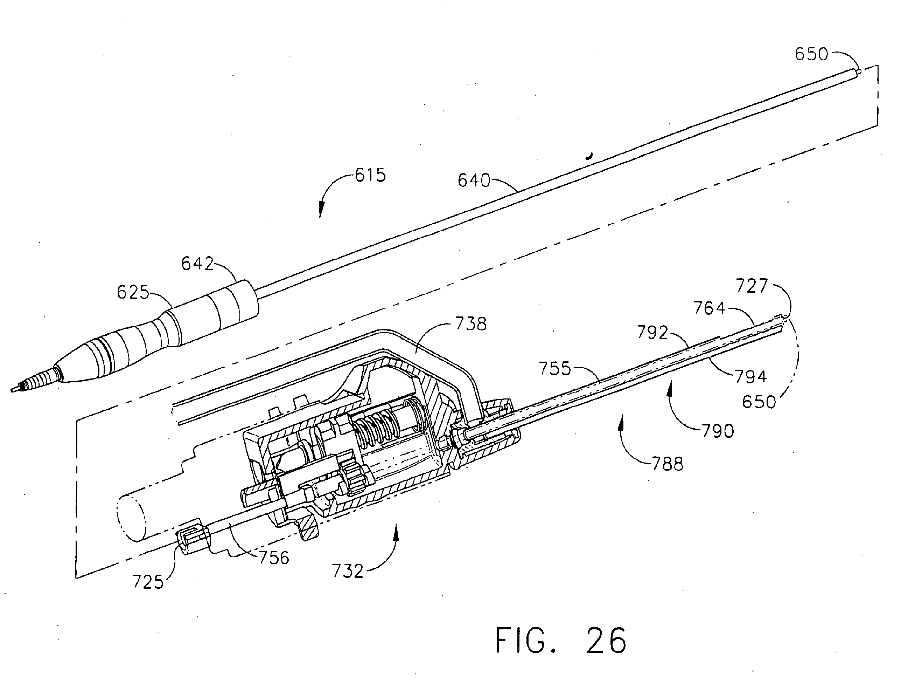

A generally continuous passageway 724 extends proximally through handpiece assembly 730 from a proximal opening 726 in probe assembly 732 to a distal bore 727 at a distal end of biopsy probe 788. Side aperture 764 on biopsy probe 788 connects with continuous passageway 724. Passageway 724 is best shown in FIG. 26 and will be described in greater detail later. Proximal opening 726 of passageway 724 is positioned to receive either the ultrasonic penetration system 615 or a second axial tube 740 using vacuum to deliver tissue samples to a tissue storage assembly 562 connected to the control module 746 and vacuum source 736.

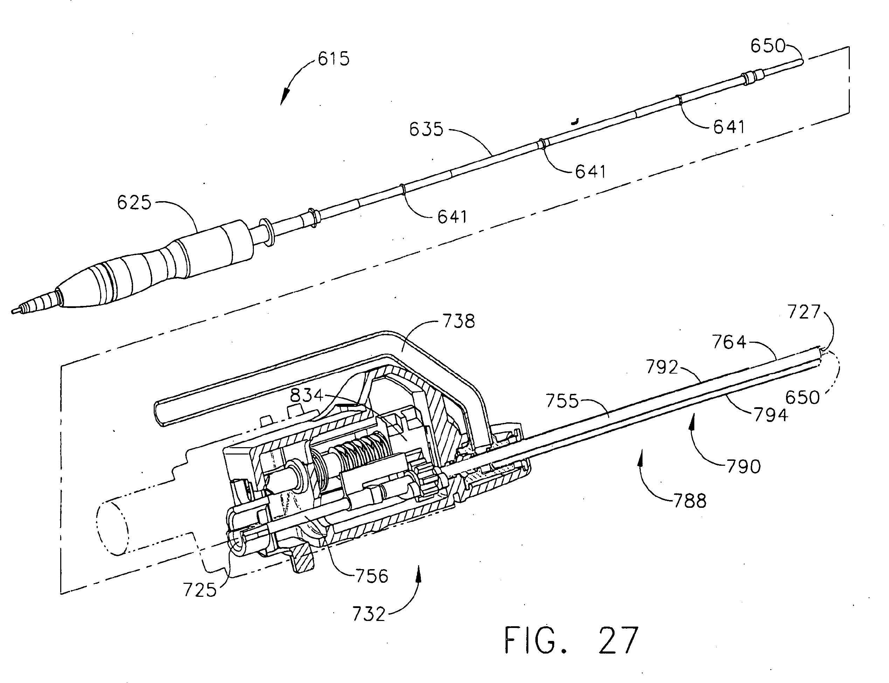

FIGURES 26 and 27 show a cross sectional view of the biopsy handpiece assembly 732 with an outline view of a detachable holster 734. A tubular cutter 755 is rotatably and slidably located in upper cutter lumen 792 of a metal cannula 790 of needle probe 788 and in handpiece assembly 732. Spur gearing is provided to rotate cutter 755 and spiral gearing moves cutter 755 proximally and distally. Handle assembly 626 of ultrasonic penetration system 615 nestles below detachable holster 734 (not shown). Generally continuous passageway 725 is formed from rear lumen 756, the bore of hollow cutter 755 and upper cutter lumen 792. A vacuum port or lower vacuum lumen 794 is connected by an opening to upper cutter lumen 792 to draw tissue into side aperture 764.

In FIG. 26, a first variant of the ultrasonic penetration system 615 is shown ready for insertion into continuous passageway 725, with an outer sleeve 640 held on by cap 642. Insertion into of the first variant of the ultrasonic penetration system 615 uses the outer sleeve 640 to block the side aperture 764 and exposes distal tip 650 through a distal bore 727.

FIG. 27 shows a second variant of the ultrasonic penetration system 615 ready for insertion into handpiece assembly 730 with the outer sleeve 640 and cap 642 removed and vibrating member 635 exposed Insertion of the second variant into biopsy handpiece assembly 730, leaves the side of vibrating member exposed through side aperture 764. For this variant, tissue contact through the side aperture 764 is blocked by moving the cutter 755 of the probe assembly 732 distally. The isolators 641 on the vibrating member 640 prevent the vibrating member from contacting inner wall of cutter 755 which forms part of continuous passageway 725. When the ultrasonic penetration system 615 is fully inserted into the biopsy handpiece 730, the distal tip 650 extends through a distal bore 727 (FIG. 26) of the biopsy probe 788. When activated, distal tip 650 of the handheld surgical system penetrates and tunnels through tissue.

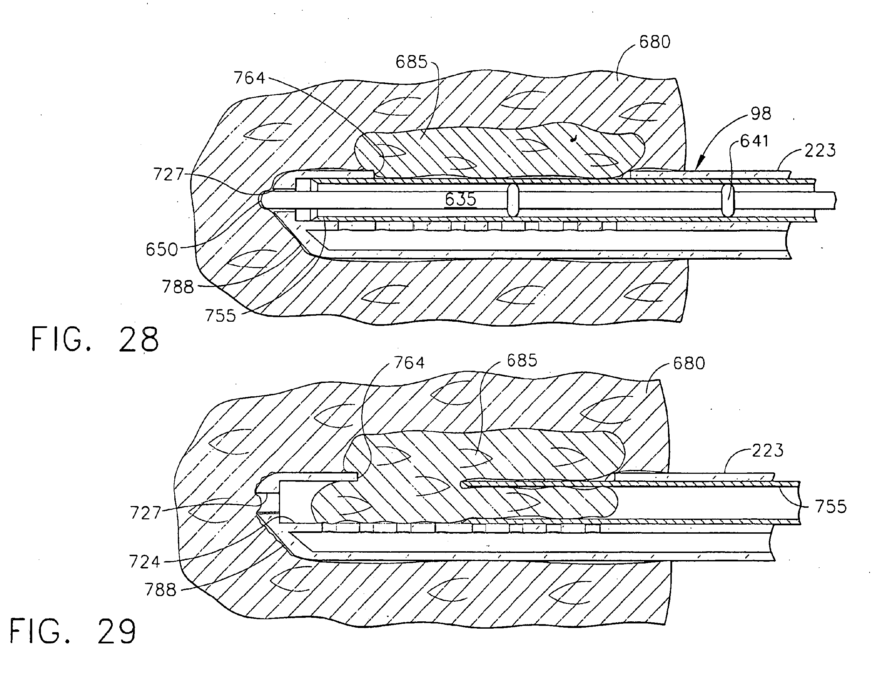

FIG. 28 shows the second variant of the ultrasonic penetration system 615 as it penetrates breast tissue 680. An energized distal tip 650 is extending through an open distal bore 727 at a distal end of biopsy needle 788. The distal tip 650 is shown tunneling through a breast 680 and tumor 685 creating a passageway therethrough. Outer tubular cutter 755 blocks side aperture 764 of biopsy needle 788 and prevents unwanted tissue contact with vibrational member 635. Vibrational member 635 has isolators 641 attached thereto to vibrationally isolate member 635 from contact with inner walls of tubular cutter 755 and rear lumen 756.

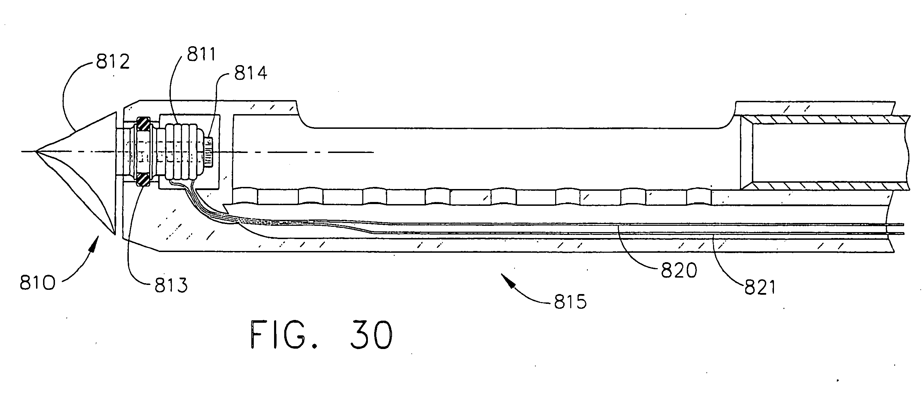

In FIG. 29, the energized distal tip 650 has tunneled into breast tissue 680 to position side aperture 376 with tumor 685 and the surgeon is ready to turn off the ultrasonic power, pull the ultrasonic penetration system 615 from the generally continuous passageway 725 and out of probe assembly 532. Tubular cutter 755 is moved to block side aperture 764 preventing tissue contact with vibrating member 635 tissue contact. As shown in FIG. 30 once the generally continuous passageway 725 is unblocked by removal of vibrating member 635, the second axial tube 540 can be attached to generally continuous passageway 725 (FIG. 15). Vacuum can now be applied to draw tissue into side aperture 764, and tubular cutter 755 moved distally to sever tumor 685. As vacuum is applied, the hard tumor 685 is sucked into the side aperture 564 and breast tissue 680 into the open distal bore 726 at a distal end of biopsy needle 788. The open distal 726 bore is deliberately sized to allow passage of the distal tip 650 of vibrational member 635, yet minimize the size of the open distal bore 726 so that it can be easily sealed with breast tissue 680 when vacuum is applied. Once severed, tissue samples 687 can be drawn from generally continuous passageway 725, down second axial tube 740, and into tissue storage assembly 562.

FIG. 30 discloses an alternate embodiment of the energy based tissue penetrating system attached to a distal end of a tissue biopsy system. In FIG. 29, an acoustic assembly 810 having one or more proximal piezoelectric elements 811 attached to a distal blade 812 is attached to a distal end of a biopsy probe 815. Piezoelectric elements 811 expand and contract in response to the electrical signals, thereby converting the electrical energy into mechanical motion to vibrate distal blade 812. The mechanical motion result in longitudinal waves of vibrational energy that propagate through the acoustic assembly 810 in an acoustic standing wave to vibrate the acoustic assembly 810 and distal blade 812 at a selected frequency and amplitude. A distal isolator 813 mounts accoustic assembly 810 in the distal end of a biopsy probe 815 and a distal fastener 814 applies preload to the elements of the acoustic assembly 810. A pair of first and second wires 820, 821 can extend longitudinally along biopsy probe 815 to electrically couple piezoelectric elements 811 to generator 620.

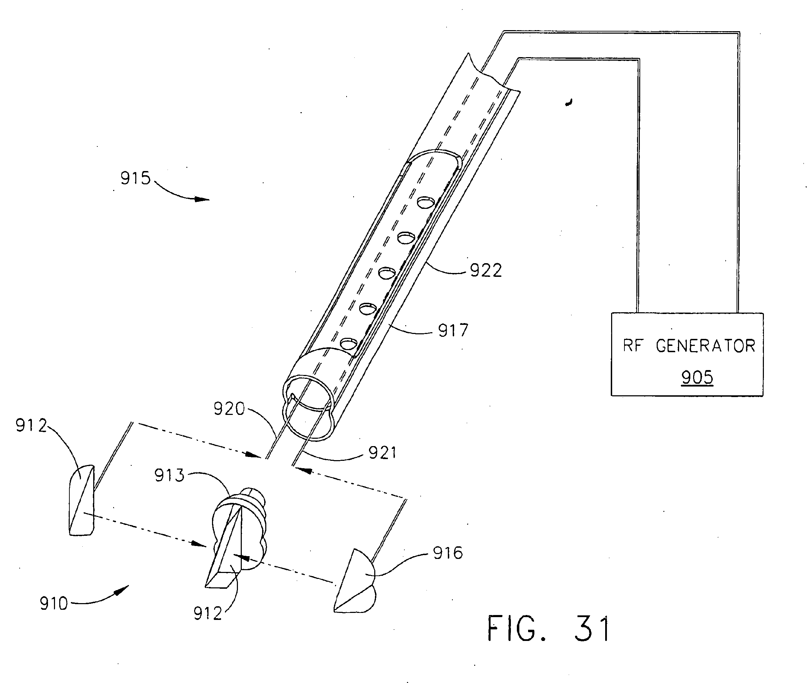

FIG. 31 discloses an exploded alternate embodiment of an energy based tissue penetration system attached to a distal end of the tissue biopsy system. For this embodiment, an RF generator 930 delivers RF bipolar energy to a RF probe assembly 910. A first pole wire 920 is operably connected to first pole electrode 912 located at a distal tip of RF probe assembly 910. First pole wire 920 is also operably connected to a first pole of generator 930. A second pole wire 921 is operably connected to a second pole electrode 921 and second pole electrode 921 and to a second pole of RF generator 930. First pole electrode 912 and second pole electrode 921 mount on an insulator 913 made from but not limited to , by way of example, a ceramic. Insulator 913 mounts on a shaft 922 of probe assembly 910. Energizing generator 930 with the appropriate cutting wave form enables the probe assembly 910 to become a RF Bipolar tunneling device. Alternately, switching to a coagulation wave form at generator 30 enables probe assembly 910 to become a RF bipolar cauterization device. If desired, a blended waveform or waveform of any shape could be used.

At least one sensor could be added near to a tissue probe to measure tissue properties as tissue is being penetrated with an energy delivery system. The measurement of tissue properties could provide feedback to a generator controller to alter the energy delivery. By way of example, a temperature sensor could be located on the RF probe 910 and provide real time information to alter energy delivery as the probe 910 tunnels into tissue. Additionally, by way of example, the temperature sensor could be used to monitor tissue effects with an ultrasonic tissue penetration system

Having shown and described various embodiments of the present invention, further adaptations of the methods and systems described herein may be accomplished by appropriate modifications by one of ordinary skill in the art without departing from the scope of the present invention. Several of such potential modifications have been mentioned, and others will be apparent to those skilled in the art. For instance, the examples, embodiments, geometries, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

While preferred embodiments of the present invention have been shown and described herein, it will be obvious to those skilled in the art that such embodiments are provided by way of example only. Numerous variations, changes, and substitutions will now occur to those skilled in the art without departing from the invention. Accordingly, it is intended that the invention be limited only by the scope of the appended claims.

## Claims

1. A biopsy device for penetrating and removing tissue comprising:
an outer cannula (223) having a lateral tissue receiving aperture (226);
a vibrating member (635) disposed within, and retractable from, said outer cannula and having a distal tip (650) exposed at a distal end of said outer cannula, said distal tip configured to penetrate tissue; and
**characterised by** an inner sleeve (640) between said vibrating member (635) and said cannula (223), said inner sleeve (640) exposing said distal tip (650) of said vibrating member (635) and blocking said lateral tissue receiving aperture (226) of said outer cannula (223), wherein the distal end of said outer cannula does not comprise a surgical sharp.

2. The biopsy device of claim 1, wherein said vibrating member (635) vibrates at ultrasonic frequencies.

3. The biopsy device of claim 1, wherein said vibrating member (635) and said inner sleeve (640) have at least one isolation element (641) extending therebetween, said isolation element operably isolating said vibrating member (635) from said inner sleeve (640).

4. The biopsy device of claim 1, wherein said vibrating member (635) and said inner sleeve (640) are insertable and removeable within said outer cannula (223), said vibrating member (635) and said inner sleeve (640) maintaining position relative to each other during insertion and removal.

5. The biopsy device of claim 1, wherein said inner sleeve (640) is removably attached about said vibrating member (635).

6. The biopsy device of claim 1, wherein at least a portion of a cross section of said inner cannula (640) is arcuate.

7. The biopsy device of claim 1, wherein said distal tip (650) has at least one surface, said at least one surface selected from the group of concave, convex, angled, arcuate, flat, spherical, conical, and sharp.

8. The biopsy device of claim 1, wherein said distal tip (650) ablates tissue.

9. The biopsy device of claim 1, wherein said vibrating member (635) causes hemostasis at wound sites.

10. The biopsy device of claim 1, where at least one of said outer cannula (223), vibrating member (635), and inner sleeve (640) is MRI compatible.

## Patentansprüche

1. Biopsievorrichtung zum Durchdringen und Entfernen von Gewebe, die aufweist:
eine äußere Kanüle (223) mit einer seitlichen gewebeaufnehmenden Öffnung (226);
ein vibrierendes Element (635), das innerhalb der äußeren Kanüle angeordnet und aus dieser zurückziehbar ist und eine distale Spitze (650) hat, die an einem distalen Ende der äußeren Kanüle freiliegt, wobei die distale Spitze dazu ausgelegt ist, Gewebe zu durchdringen; und
**gekennzeichnet durch** eine innere Hülse (640) zwischen dem vibrierenden Element (635) und der Kanüle (223), wobei die innere Hülse (640) die distale Spitze des vibrierenden Elementes (635) freilegt und die seitliche gewebeaufnehmende Öffnung (226) der äußeren Kanüle (223) blockiert, wobei das distale Ende der äußeren Kanüle keine chirurgische Schärfe aufweist.

2. Biopsievorrichtung nach Anspruch 1, bei der das vibrierende Element (635) bei Ultraschallfrequenzen schwingt.

3. Biopsievorrichtung nach Anspruch 1, bei der das vibrierende Element (635) und die innere Hülse (640) wenigstens ein Isolierelement (641) haben, das sich zwischen ihnen erstreckt, wobei das Isolierelement das vibrierende Element (635) betrieblich von der inneren Hülse (640) isoliert.

4. Biopsievorrichtung nach Anspruch 1, bei der das vibrierende Element (635) um die innere Hülse (640) in die äußere Kanüle (223) einsetzbar und aus dieser entfernbar sind, wobei das vibrierende Element (635) und die innere Hülse (640) während des Einführens und des Entfernens ihre relative Position zueinander beibehalten.

5. Biopsievorrichtung nach Anspruch 1, bei der die innere Hülse (640) entfernbar um das vibrierende Element (635) herum angebracht ist.

6. Biopsievorrichtung nach Anspruch 1, bei der wenigstens ein Teil eines Querschnitts der inneren Kanüle (640) bogenförmig ist.

7. Biopsievorrichtung nach Anspruch 1, bei der die distale Spitze (650) wenigstens eine Fläche hat, wobei die wenigstens eine Fläche aus der Gruppe aus konkav, konvex, gewinkelt, gebogen, flach, sphärisch, konisch und scharf ausgewählt ist.

8. Biopsievorrichtung nach Anspruch 1, bei der die distale Spitze (650) Gewebe abträgt.

9. Biopsievorrichtung nach Anspruch 1, bei der das vibrierende Element (635) die Blutstillung an verletzten Stellen hervorruft.

10. Biopsievorrichtung nach Anspruch 1, bei der wenigstens eines aus äußerer Kanüle (223), vibrierendem Element (635) und innerer Hülse (640) mit der MRI verträglich ist.

## Revendications

1. Dispositif de biopsie pour pénétrer dans un tissu et le retirer, comprenant :
une canule extérieure (223) comportant une ouverture de réception de tissu latérale (226) ;
un élément vibrant (635) disposé à l'intérieur de ladite canule extérieure, et pouvant être rétracté de celle-ci, et comportant une pointe distale (650) exposée à une extrémité distale de ladite canule extérieure, ladite pointe distale étant configurée de façon à pénétrer dans un tissu ; et
**caractérisé par** un manchon intérieur (640) entre ledit élément vibrant (635) et ladite canule (233), ledit manchon intérieur (640) exposant ladite pointe distale (650) dudit élément vibrant (635) et bouchant ladite ouverture de réception de tissu latérale (226) de ladite canule extérieure (223), l'extrémité distale de ladite canule extérieure ne comprenant pas de tranchant chirurgical.

2. Dispositif de biopsie selon la revendication 1, dans lequel ledit élément vibrant (635) vibre à des fréquences ultrasoniques.

3. Dispositif de biopsie selon la revendication 1, dans lequel ledit élément vibrant (635) et ledit manchon intérieur (640) comportent au moins un élément d'isolement (641) s'étendant entre ceux-ci, ledit élément d'isolement isolant de façon fonctionnelle ledit élément vibrant (635) dudit manchon intérieur (640).

4. Dispositif de biopsie selon la revendication 1, dans lequel ledit élément vibrant (635) et ledit manchon intérieur (640) peuvent être insérés et retirés à l'intérieur de ladite canule extérieure (223), ledit élément vibrant (635) et ledit manchon intérieur (640) conservant leur position l'un par rapport à l'autre durant l'insertion et le retrait.

5. Dispositif de biopsie selon la revendication 1, dans lequel ledit manchon intérieur (640) est fixé de façon amovible autour dudit élément vibrant (635).

6. Dispositif de biopsie selon la revendication 1, dans lequel au moins une partie d'une section transversale de ladite canule intérieure (640) est en forme d'arc.

7. Dispositif de biopsie selon la revendication 1, dans lequel ladite pointe distale (650) comporte au moins une surface, ladite au moins une surface étant choisie dans le groupe comprenant les formes concave, convexe, en angle, en arc, plate, sphérique, conique et aiguë.

8. Dispositif de biopsie selon la revendication 1, dans lequel ladite pointe distale (650) réalise une ablation de tissu.

9. Dispositif de biopsie selon la revendication 1, dans lequel ledit élément vibrant (635) provoque une hémostase en des sites blessés.

10. Dispositif de biopsie selon la revendication 1, dans lequel au moins l'un parmi ladite canule extérieure (223), ledit élément vibrant (635) et ledit manchon intérieur (640) est compatible avec l'imagerie à résonance magnétique.
